# EUROPEAN PATENT APPLICATION

(11) **EP 4 700 794 A1**
(43) Date of publication of application: **25.02.2026**
(21) Application number: 25197397.0
(22) Date of filing: 21.08.2025
(51) Int. Cl.: G16H 50/50, G16H 20/10, G16H 20/60, G16H 50/20, G16H 50/30

(54) **REAL-TIME DELIVERY OF DYNAMIC, PERSONALIZED DIGITAL THERAPIES TO USERS FOR ADDRESSING OBESITY CONDITIONS USING PHENOTYPE-DRIVEN DIGITAL TWIN MODELS**

(30) Priority: 22.08.2024 US 202463685858 P
(71) Applicant: Click Therapeutics, Inc., New York, NY 10013 (US)
(72) Inventor: DAHLEM, Markus, New York, 10013 (US); ADLER, Samantha, New York, 10013 (US)
(74) Representative: Mewburn Ellis LLP

(57) **Abstract**

Provided herein are systems and methods for presenting interventions to address obesity in users related to using digital twin model. A computing system may receive measurements of a metabolic system of a user. The computing system can then apply the measurements to a function to generate an output. The computing system can update, using the output from the function, at least one of a plurality of weights of a digital twin of the user. The computing system can generate using the digital twin, a metric indicative of an obesity condition in the metabolic system of the user. The computing system can identify an intervention for the obesity condition based on the metric and provide to a user device, an instruction identifying the intervention for the metabolic system of the user. The computing system can improve efficacy of the medication that the user is taking in concurrence to address their condition.

## Description

### CROSS-REFERENCE TO RELATED APPLICATIONS

This application claims priority to U.S. Provisional Application No. 63/685,858, filed August 22, 2024, entitled "REAL-TIME DELIVERY OF DYNAMIC, PERSONALIZED DIGITAL THERAPIES TO USERS FOR ADDRESSING OBESITY CONDITIONS USING PHENOTYPE-DRIVEN DIGITAL TWIN MODELS," the contents of which is incorporated by reference in its entirety.

### BACKGROUND

Obesity is characterized by an excess accumulation of adipose tissue, leading to increased body weight and associated health risks. This condition may arise from a diverse array of underlying factors, such as medical conditions and lifestyle factors. For example, specific underlying factors that may give rise to obesity include metabolic disorders (e.g., hypothyroidism or diabetes), endocrine dysfunction (e.g., Cushing's Syndrome, polycystic ovary syndrome, or growth hormone deficiency disorders), genetic predisposition (e.g., Prader-Willi syndrome), psychological conditions (e.g., binge eating disorder, depression, or anxiety), medication abuse (e.g., antidepressants or antipsychotics), lifestyle choices (e.g., unhealthy diet or irregular eating patterns), developmental issues (e.g., gestational diabetes), or sleep-related issues (e.g., sleep deprivation), among others. At the molecular level, for individuals with obesity, fat cells (known as adipocytes) increase in size or number, and as these cells expand can become dysfunctional. In addition, at the physiological level, obesity results from an imbalance between energy intake and energy expenditure, among other issues.

Obesity presents a challenge, impacting the health, social well-being, and mental health of those individuals that are affected. Individuals with obesity are at heightened risk for a range of physical health complications, including hypertension, sleep apnea, asthma, and an increased susceptibility to various forms of cancer, such as breast, colon, endometrial, and pancreatic cancers. Additionally, the psychological burden, such as body image issues, social stigma, and a diminished quality of life due to reduced mobility or chronic pain, further complicates the challenge of obesity. Body image issues often lead to diminished self-esteem and an increased risk of depression and anxiety. Moreover, the overall quality of life for those with obesity can be severely compromised. Reduced mobility could also be an issue, where the physical burden of excess weight limits an individual's ability to engage in everyday activities, leading to chronic pain, joint problems, and an increased risk of falls and injuries.

Weight loss therapies can be used to facilitate weight loss, and can include, for example, a prescribed regimen for diet changes, increased physical activity, or increased sleep, among other. Achieving weight loss, however, can be difficult due to ineffective regimens. In particular, individual metabolic variations play a crucial role in the effectiveness of weight loss therapies. For instance, a regimen focused on increasing sleep time or reduced calorie intake may yield significant results for one person while having minimal impact on another. Failing to take into account dynamically changing physiological measurements of the individual, user activity data, and other relevant information can lead to ineffective weight loss therapies, and ultimately little to no improvement in health outcomes.

Adherence to weight loss therapies is a key determinant of success. However, when a regimen leads to limited or no improvement, individuals may struggle to maintain their progress and achieve their weight loss goals. Existing weight loss and obesity regimens fail to account for the challenges associated with maintaining individual accountability and decreased engagement with treatment interventions. Specifically, there is a need for a digital therapeutic application that addresses these concerns by providing a real time assessment and progressive feedback to a user for the purpose of ameliorating obesity and metabolic dysfunction.

### SUMMARY

To address these and other technical problems with suboptimal weight loss outcomes and lack of adherence to prescribed therapies, the digital therapeutic application herein can leverage a phenotype-driven digital twin model that integrates biological processes, employs real-time data assimilation. This can facilitate generating personalized optimization of combined pharmacological and digital therapeutic interventions as well as accurately modeling the complex interplay of biological, behavioral, and environmental factors influencing an individual's response to various interventions. Presented herein are systems and methods for generating interventions to address obesity and address weight loss by using digital twins to simulate the metabolic systems of users based on various measurements. Specifically, the digital therapeutic application described herein leverages signal processing and filter techniques for state estimation and prediction to create a digital twin to model the metabolic system of a particular user, simulating the user's unique biological processes related to obesity and energy imbalance. In particular, the digital twin can simulate an energy balance of a body using at least four major domains of glucose excretion (GE), energy expenditure (EE), physical activity (PA), and energy intake (EI), among others. The digital therapeutic application described may incorporate a Kalmin filter to simulate the user's metabolic health and then generate interventions based on the user's unique metabolic signature.

There are several advantages for the users of a digital therapeutic application detailed herein in leveraging the digital twin to select and provide appropriate interventions for the user. For one, the digital therapeutics application addresses the lack of personalized solutions that effectively integrate biological data. The application uses sophisticated machine learning techniques to predict treatment outcomes by iteratively updating its state estimates and combining forecasts visualizations. Further, the machine learning model continuously updates the digital twin based on real-time measurements, ensuring precision and accuracy in reflecting the individual's physiological state. Thus, the digital therapeutic application is able to utilize information on the user's unique metabolic signature to provide specifically tailored interventions for the treatment of obesity.

For another, by predicting individualized responses to a combination of drug and device-based weight loss therapies, the digital therapeutic application seamlessly integrates pharmaceutical interventions with advanced digital interventions. The digital twin can be used to simulate the impact of various interventions, allowing the machine learning model to either select the most effective treatment strategy or present options that align with the individual's goals. Unlike other approaches that do not incorporate real-time data, the digital twin uses recursive data assimilation, considering all relevant factors that influence energy balance and metabolic health. The use of the digital twin enables the creation of highly personalized treatment strategies, optimizing the integration of pharmacological and digital therapeutic interventions.

In addition, the digital therapeutic application provides visual motivators to encourage adherence to treatment plans. For instance, leveraging the forecasting capabilities of the Kalman filter, the system can generate AI-driven avatars that depict potential body composition changes based on the selected intervention. These visualizations serve as powerful tools for motivating users to stay committed to their weight loss goals. By continuously updating and optimizing the treatment plan through real-time feedback, the digital twin ensures that interventions remain effective and aligned with the user's evolving needs.

All in all, the digital therapeutic application addresses the lack of accurate, personalization integration of biological data by providing an individualized model that dynamically predicts individual responses to various treatment strategies. The application offers a precise and tailored approach to weight management that aligns with the specific needs and physiological profiles of each patient. Through this integration of digital and pharmacological solutions, the treatment of obesity and related metabolic disorders is immensely enhanced, driving better outcomes and improving overall patient care.

Aspects of the present disclosure is directed towards systems and methods for selecting treatments for an obesity condition in a user. The system can include one or more processors, configured to receive one or more measurements of a metabolic system of the user over a measured time period. The one or more processors can be further configured to apply the one or more measurements to a function to generate an output for a subsequent time period. The one or more processors can be further configured to update, using the output from the function, at least one of a plurality of weights of a digital twin of the user, the plurality of weights corresponding to the metabolic system of the user. The one or more processors can be further configured to generate, using the digital twin, a metric of the metabolic system of the user for the subsequent time period. In some aspects, the metric is indicative of the presence of the obesity condition in the user. The one or more processors can be further configured to identify an intervention for the obesity condition based on the metric. The one or more processors can be further configured to provide to a user device, an instruction identifying the intervention for the metabolic system of the user.

In various implementations, the one or more processors are configured to provide an instruction for administering the intervention for the obesity condition to the user. The one or more processors can be further configured to update the digital twin with information on the administration of the intervention, and generate using the digital twin, a second metric of the metabolic system based on the information. The one or more measurements of the metabolic system can include at least one of: glucose excretion, an evaluation of an infradian or circadian rhythm, energy expenditure, physical activity, hormone levels, body weight, body fat percentage, a genetic marker, an evaluation of the gut microbiome, or energy intake. The intervention can be selected, by the one or more processors from one or more of the following: a weight loss medication, physical activity, a reduction in energy intake, food choices, cognitive training, behavioral therapy, an optimal mealtime, an exercise routine, and a surgical intervention. The weight loss medication can be selected from one or more of a GLP-1 receptor agonist, GIP receptor agonists, a lipase inhibitor, an amphetamine, an antidepressant, an opioid medication, and a melanocortin receptor agonist. The weight loss medication can be a GLP-1 receptor agonist selected from one or more of semaglutide, liraglutide, exenatide, and dulaglutide, or a GIP receptor agonist comprising tirzepatide.

In various implementations, the one or more processors may update at least one of the plurality of weights of the digital twin responsive to changes in the one or more measurements applied to the filter. The updating, by the one or more processors, can be in real-time and responsive to the changes in the one or more measurements. The updating, by the one or more processors, can be responsive to any one of the following: the user's activity level, meal timing, the time of day, the user's circadian rhythms, and sleep and/or wake timing. The function can be a filter. The filter can include at least one of a Kalman filter, a particle filter, a sliding window filter, an information filter, an extended Kalman filter, or an unscented Kalman filter. In some aspects, the filter may utilize a Monte Carlo simulation. The one or more processors can be configured to simulate a second metric indicating a condition of the metabolic system of the user in response to the introduction of the intervention. The second metric can include a simulation of the user's energy balance, weight loss trajectory, energy intake, energy output, physical activity, glucose excretion, a simulation of a body composition change, a metabolic rate, a weight loss prediction, glucose fluctuations, or energy expenditure.

In various implementations, the one or more processors may generate a visual output of the second metric to the user device. The visual output can be updated, by the one or more processors, based on the preferences of the user. The preferences of the user can be selected from: a plurality of metrics of metabolic health, graphical outputs, and the visual format of the interface. The visual output can be an avatar of the digital twin based on a body composition change resulting from the simulated introduction of the intervention, an AI generated image of the digital twin, an infographic, a graphical output, or a metric of metabolic health. The digital twin can include a corresponding plurality of scores determined by the one or more processors, by the one or more measurements of the user. The system can include the one or more processors configured to generate of the metric of metabolic health further comprises determining the metric based on at least one of: (i) an average of the plurality of scores, (ii) a weighted combination of the plurality of scores, or (iii) a comparison with a dataset comprised of a second plurality of scores and generate a plurality of treatment strategies. The user can select a treatment strategy from the plurality of treatment strategies.

Another aspect of the present disclosure is directed towards systems or methods of ameliorating an obesity condition in a user in need thereof. The method can include administering, to the user, a treatment comprising a digital therapeutic. The digital therapeutic can include receiving one or more measurements of a metabolic system of the user over a measured time period and applying the one or more measurements to a function to generate an output for a subsequent time period. The digital therapeutic can also include updating, using the output from the function, at least one of a plurality of weights of a digital twin of the user, the plurality of weights corresponding to the metabolic system of the user. The digital therapeutic can include generating, using the digital twin, a metric of the metabolic system of the user for the subsequent time period. The metric of the metabolic system of the user may indicate the presence of the obesity condition in the user. The digital therapeutic can include identifying an intervention for the obesity condition based on the metric.

In various implementations, the digital therapeutic can include obtaining a second metric of the condition of the metabolic system of the user subsequent to administering the digital therapeutic. The method can include administration of the digital therapeutic, to the user, can result in (i) a decrease from the first metric by a first predetermined margin or (ii) an increase from the first metric by a second predetermined margin. The method can include following administration of the digital therapeutic, to the user, the second metric can be (i) decreased from the first metric by a first predetermined margin or (ii) increased from the first metric by a second predetermined margin. The method can further include administering the intervention for the treatment of the obesity condition to the user. The digital therapeutic can further include determining a second intervention for the obesity condition in the user. The method can further include administering the second intervention for the obesity condition to the user. The first metric of the obesity condition can be based on a plurality of weights of the digital twin.

In various implementations, the digital therapeutic further includes simulating, by one or more processors, a third metric of the metabolic system of the user based on the administration of the intervention to the user. The digital therapeutic further includes generating a visual output of the third metric to a user device. The one or more measurements of the metabolic system can include at least one of: glucose excretion, an evaluation of an infradian or circadian rhythm, energy expenditure, physical activity, hormone levels, body weight, body fat percentage, a genetic marker, an evaluation of the gut microbiome, or energy intake. The intervention can be selected from one or more of the following: a weight loss medication, physical activity, a reduction in energy intake, food choices, cognitive training, behavioral therapy, an optimal mealtime, an exercise routine, and a surgical intervention. The weight loss medication can be selected from one or more of a GLP-1 receptor agonist, GIP receptor agonists, a lipase inhibitor, an amphetamine, an antidepressant, an opioid medication, and a melanocortin receptor agonist. The weight loss medication can be a GLP-1 receptor agonist selected from one or more of semaglutide, liraglutide, exenatide, and dulaglutide, or a GIP receptor agonist comprising tirzepatide.

In various implementations, the user is receiving treatment for obesity. The user can be suffering from diabetes, high blood pressure, hypercholesterolemia, fatigue, excess body fat, psychological issues, snoring, shortness of breath, or physical impairments. The intervention can be administered over a period of 1 day, 5 days, 1 week, 2 weeks, 1 months, 3 months, 6 months, 1 year or longer. The user can be provided with a wearable technology configured to obtain one or more of the physiological measurements. The user can have a BMI greater than or equal to 25, greater than or equal to 30, greater than or equal to 32, or greater than or equal to 35. The user can have a body fat percentage greater than 20%, greater than 25%, or greater than 30%. Administration of the digital therapeutic can result in a reduction of BMI, body fat percentage, blood glucose, or body weight.

### BRIEF DESCRIPTION OF THE DRAWINGS

The foregoing and other objects, aspects, features, and advantages of the disclosure will become more apparent and better understood by referring to the following description taken in conjunction with the accompanying drawings, in which:
FIG. 1 depicts a block diagram of a system for presenting interactive sessions to address functional impairment in users in accordance with an illustrative embodiment.
FIGs. 2A and 2B depicts a block diagram for a process to provide a session to a user to generate an intervention, in accordance with an illustrative embodiment.
FIG. 2C depicts a block diagram of an architecture for the digital twin model and a Kalman filter, in accordance with an illustrative embodiment.
FIG. 3 depicts a process used by a model to generate a digital twin of the user, in accordance with an illustrative embodiment.
FIG. 4 depicts a block diagram for a process to generate the intervention using a Kalman filter in accordance with an illustrative embodiment.
FIG. 5 depicts screenshots of sets of example user interfaces for generating the intervention, in accordance with an illustrative embodiment.
FIG. 6 depicts a flow diagram of a method for identifying an intervention for the user to address obesity in accordance with an illustrative embodiment.
FIG. 7 depicts a flow diagram of a method of ameliorating obesity in users via administration of digital therapeutics in accordance with an illustrative embodiment.
FIG. 8 illustrates an example study schedule of an efficacy study for the CT-100 mobile app.
FIG. 9 illustrates an example study schedule of an engagement study for a CT-100 mobile app.
FIG. 10 is a block diagram of a server system and a client computer system in accordance with an illustrative embodiment.

### DETAILED DESCRIPTION

For purposes of reading the description of the various embodiments below, the following enumeration of the sections of the specification and their respective contents may be helpful:
Section A describes systems and methods for providing instructions for an intervention for an obesity condition in a user.
Section B describes systems and methods for ameliorating obesity in users via administration of digital therapeutics.
Section C describes a network and computing environment which may be useful for practicing embodiments described herein.

### A. Systems and Methods for Providing Instructions for an Intervention for an Obesity Condition in a User

Obesity presents a challenge, impacting the health, social well-being, and mental health of those individuals that are affected. Individuals with obesity are at heightened risk for a range of physical health complications, including hypertension, sleep apnea, asthma, and an increased susceptibility to various forms of cancer, such as breast, colon, endometrial, and pancreatic cancers.

The digital therapeutic application described herein can receive measurements of a metabolic system of the user over a measured time period. The measured time period can be indicated by the digital therapeutic application to the user. The one or more measurements may include values input by the user, blood test results, or wearable technology data, among others. As the measurements are received, the application can apply the one or more measurements to a function to generate an output for a subsequent time period. The function can be a part of a machine learning model, where a Kalman filter is used for state estimation and forecasting alongside other machine learning techniques. As the one or more measurements are applied, the at least one of a set of weights of a digital twin of the user can be updated using output of the function. The digital twin can be a part of a machine learning model. The set of weights can each correspond to an aspect of the metabolic system of the user.

By using the digital twin, the application can generate a metric indicative of an obesity condition of the user. The metric can indicate a severity of the obesity condition. In generating the metric, the application can take into account the one or more measurements to alter the metric based on a possible efficacy of various intervention strategies. With the metric, the application can identify an intervention for the obesity condition. The intervention can be continuously updated based on continuously updating one or more measurements. The intervention can include one or more of a weight loss medication, physical activity, a reduction in energy intake, food choices, cognitive training, behavioral therapy, an optimal mealtime, an exercise routine, and a surgical intervention. The application can then provide an instruction to a user device of the user identifying the intervention. Identification of the intervention can take various forms such as a set of tasks, a table, a chart, or a database, among others.

In addition, the application can provide an instruction administering the intervention to the user. For example, the instruction may include a message instructing the user to follow a physical activity plan daily, and input the physical activity. Based on information gathered by administration of the intervention, the digital twin can be updated and generate a second metric to adjust the intervention. The application may also provide a visual output when providing the intervention to the user. The visual output can be an avatar of the digital twin based on a body composition change resulting from the simulated introduction of the intervention, an AI generated image, an infographic, a graphical output, or a metric of metabolic health. The digital twin can simulate various interventions, and the application can generate and output the avatar based on body composition changes determined by the digital twin.

In this manner, the application may use the incoming measurements to continuously update the digital twin, and subsequently the intervention to constantly optimize the intervention to promote weight loss in the user. By taking into account the energy balance of the user, the application can generate a treatment plan that also takes in account hidden factors, such as energy expenditure per unit weight change, to tailor the treatment plan to the metabolic system of the user. The application can also take into account user preferences to generate the treatment plan and personalize a user interface to best promote adherence to the treatment plan. For example, by providing the avatar to predict how the user's appearance may change, the application can motivate adherence to a chosen treatment plan. The application can blend pharmacotherapy and digital therapy solutions to facilitate weight loss in users, while avoiding unnecessary time spent researching weight loss plans.

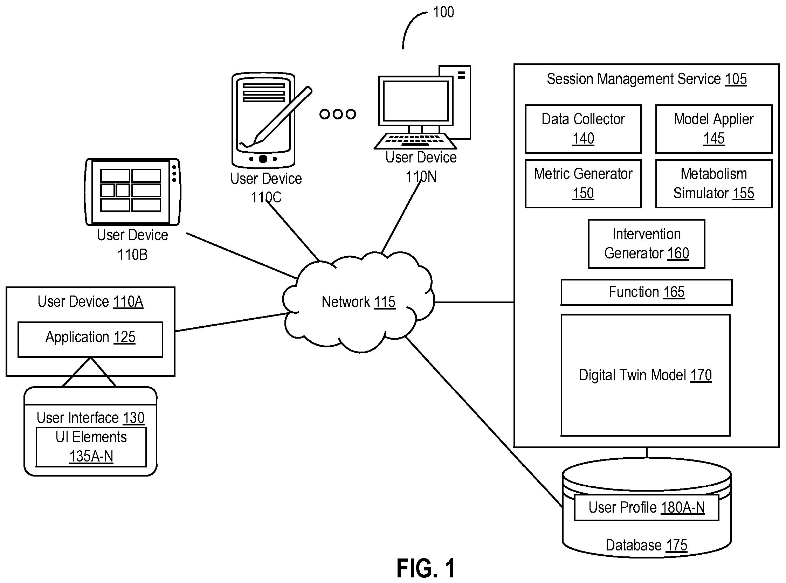

Referring now to FIG. 1, depicted is a block diagram of a system 100 for presenting interactive sessions to address obesity in users. In an overview, the system 100 may include at least one session management service 105 and a set of user devices 110A-N (hereinafter generally referred to as user devices 110), communicatively coupled with one another via at least one network 115. At least one user device 110 (e.g., the first user device 110A as depicted) may include at least one application 125. The application 125 may include or provide at least one user interface 130 with one or more user interface (UI) elements 135AN (hereinafter generally referred to as UI elements 135). The session management service 105 may include at least data collector 140, model applier 145, metric generator 150, metabolism simulator 155, intervention generator 160, at least one function 165, and at least one digital twin model 170, among others. The session management service 105 may include or have access to at least one database 175. The database 175 may store, maintain, or otherwise include one or more user profiles 180 A-N (hereinafter generally referred to as user profiles 180). The functionality of the application 125 may be performed in part on the session management service 105. Conversely, the functionality of the application 125 may also incorporate operations performed on the session management service 105. Collectively, the user device 110 and the session management service 105 may be part of a computing system to provide the application 125.

In further detail, the session management service 105 may (sometimes herein generally referred to as a service) be any computing device comprising one or more processors coupled with memory and software and capable of performing the various processes and tasks described herein. The session management service 105 may be in communication with the one or more user devices 110 and the database 175 via the network 115. The session management service 105 may be situated, located, or otherwise associated with at least one server group. The server group may correspond to a data center, a branch office, or a site at which one or more servers corresponding to the session management service 105 is situated. The session management service 105 may be situated, located, or otherwise associated with one or more of the user devices 110. Some components of the session management service 105 may be located within the server group, and some may be located within the client device. For example, the session management service 105 may operate or be situated on the user device 110, and the digital twin model 170 may operate or be situated on the server group.

Within the session management service 105, the data collector 140 may present to a user by the application 125 on respective user devices 110 data to input. The data collector 140 can then collect the data provided by the user, and the model applier 145 can receive the data. The model applier 145 can apply the data from the user to a machine learning model (e.g., the digital twin model 170) to analyze the data to determine different possible treatment modalities and generate a digital twin. The metabolism simulator 155 can simulate various interventions (e.g., treatment plans) on the digital twin, and then generate a recommended treatment for the user via the intervention generator 160.

The digital twin model 170 (sometimes referred herein as a machine learning (ML) model including possibly Kalman filter used for state estimation and forecasting alongside other machine learning techniques) can be used to determine the digital twin of the user. The architecture for the machine learning model can include, for example, a deep learning neural network (e.g., convolutional neural network architecture, a residual network, or a transformer-based architecture), a regression model (e.g., linear or logistic regression model), a random forest, a support vector machine (SVM), a clustering algorithm (e.g., k-nearest neighbors), or a Naive Bayesian model, among others and be supervised, unsupervised, or self-supervised. In general, the digital twin model 170 may have at least one input and output. The input and output may be related via a set of weights. The input may be data from the user while the output may be at least one or more interventions, digital twins, and visual outputs. The visual outputs can include an artificial intelligence (AI) generated image of the digital twin, an infographic, a graphical output, or a metric of metabolic health. In some embodiments, the digital twin model 170 can use an energy balance equation that incorporates glucose excretion (GE), energy expenditure (EE), physical activity (PA), and energy intake (EI).

The function 165 can be used to update the weights of the machine learning model architecture used to implement the digital twin model 170. The filter may include, for example, a Kalman filter, a particle filter, a sliding window filter, an information filter, an extended Kalman filter, or an unscented Kalman filter. In some aspects, the filter may utilize a Monte Carlo simulation. For example, the digital twin model 170 can also use a Kalman filter to continuously update parameters of the digital twin model 170 based on incoming, real-time input (e.g., by the user). The digital twin model 170 can estimate hidden variables of the input (e.g., energy density associated with weight change) to produce the output (e.g., the intervention).

The user device 110 (sometimes herein referred to as an end user computing device or client device) may be any computing device comprising one or more processors coupled with memory and software and capable of performing the various processes and tasks described herein. The user device 110 may be in communication with the session management service 105 and the database 175 via the network 115. The user device 110 may be a smartphone, other mobile phone, tablet computer, a wearable device (e.g., smart watch, eyeglasses), or laptop computer. The user device 110 may be used to access the application 125. In some embodiments, the application 125 may be downloaded and installed on the user device 110 (e.g., via a digital distribution platform). In some embodiments, the application 125 may be a web application with resources accessible via the network 115.

The application 125 executing on the user device 110 may be a digital therapeutics application and may provide the sessions (sometimes herein referred to as a therapy session) to address obesity conditions and diseases associated with obesity conditions. The user of the application 125 may be suffering from, diagnosed with, or at risk of a disease or disorder associated with an obesity condition. For example, the user can be suffering from diabetes, high blood pressure, hypercholesterolemia, fatigue, excess body fat, psychological issues, snoring, shortness of breath, or physical impairments, among others. The disease or disorder may include any number of disorders that cause obesity in the user. The user can have a body weight index (BMI) greater than or equal to 25, greater than or equal to 30, greater than or equal to 32, or greater than or equal to 35. The user can have a body fat percentage of greater than 20%, greater than 25%, or greater than 30%. The causes of obesity can include genetic, behavioral, environmental, physiological, and psychological factors, among others. For example, certain genetic traits can affect weight gain such as a slower metabolism and/or appetite regulation. A family history of obesity can also increase a likelihood of developing obesity based on lifestyle habits and shared genetics. In another example, metabolic conditions (e.g., diabetes), can cause weight gain such as due to increased appetite and caloric intake due to higher blood sugar levels.

Obesity can correspond to physical health effects, mental health effects, social and economic effects, and impact children of a patient with obesity. The physical health effects may include, for example, a cardiovascular disease (e.g., hypertension, heart disease, or stroke) or a metabolic disorder (e.g., diabetes or dyslipidemia), among others. The condition may impede or hinder social skills, such as facing discrimination or stigma in various social settings, such as the workplace or healthcare facilities.

The application 125 may be used to provide interventions for weight loss to the user. The interventions may be presented to the user as a result of the user inputting data (e.g., physiological measurements, lifestyle data), generating a digital twin of the user, or performing simulations of results of various interventions on the digital twin, among others. The intervention may be presented alongside a visual output of a result of following the intervention, such as an avatar of the digital twin representing a body composition change of the user. The intervention may include at least one or more of, for example, weight loss medication, physical activity, a reduction in energy intake, food choices, cognitive training, behavioral therapy, an optimal mealtime, an exercise routine, or a surgical intervention, among others. Other behaviors may cause or be related to obesity of the user. By providing the user the digital therapeutics (e.g., the intervention) through the application 125, the adverse effects of obesity can be addressed.

The user may be at least partially concurrently receiving a treatment to address the condition, or side effects of the condition, at least partially concurrently with the interventions provided by the application 125. For example, the user may be receiving treatment for obesity. The user may be receiving a treatment at least partially concurrently with a first intervention, a second intervention, a third intervention, or any combination thereof. The treatment can include a taking of a medication. The medication may be at least orally administered, intravenously administered, or topically applied. For example, for obesity, the user may include obesity medications (e.g., orlistat, phentermine-topiramate, lorcaserin, naltrexone-bupropion, liraglutide, or semaglutide), among others. For metabolic conditions (e.g., diabetes or hypothyroidism), the user may be on diabetes medications (e.g., insulin, biguanides, sulfonylureas, or meglitinides) or beta-blocker drugs (e.g., propranolol or atenolol), among others. The application 125 may increase the efficacy of the medication that the user is taking to address the condition. The treatment can include blood tests, bariatric surgery, metabolic surgery, nutritional counseling, psychological counseling, or weight management programs, among others.

The application 125 can include, present, or otherwise provide a user interface 130 including the one or more UI elements 135 to a user of the user device 110 in accordance with a configuration on the application 125. The UI elements 135 may correspond to visual components of the user interface 130, such as a command button, a text box, a check box, a radio button, a menu item, and a slider, among others. In some embodiments, the application 125 may be a digital therapeutics application and may provide one or more sessions (sometimes referred to herein as a therapy session) via the user interface 130 for addressing functional impairments in users.

The database 175 may store and maintain various resources and data associated with the session management service 105 and the application 125. The database 175 may include a database management system (DBMS) to arrange and organize the data maintained thereon. The database 175 may be in communication with the session management service 105 and the one or more user devices 110 via the network 115. While running various operations, the session management service 105 and the application 125 may access the database 175 to retrieve identified data therefrom. The session management service 105 and the application 125 may also write data onto the database 175 from running such operations.

Such operations may include the maintenance of the user profile 180 (sometimes herein referred to as a subject profile). The user profile 180 can include information pertaining to a condition of a user, as described herein. For example, the user profile 180 may include information related to the severity of the condition, occurrences of the condition (such as occurrences of symptoms associated with the condition affecting the cognitive functioning of the user), medications or treatments the user takes for the condition, and/or a duration of the condition, among others. The user profile 180 can be updated responsive to a schedule, periodically (e.g., daily, weekly), responsive to a change in user information (e.g., input by the user via the user interface 130 or learned from the user device 110), or responsive to a clinician (e.g., a doctor or nurse) addressing the user's condition, among others.

The user profile 180 can store and maintain information related to a user of the application 125 through user device 110. Each user profile 180 may be associated with or correspond to a respective user of the application 125. The user profile 180 may contain or store information for each session performed by the user. The information for a session may include various parameters, actions, the images, prompts, or selections or actions of previous sessions performed by the user and may initially be null. The user profile 180 can enable streamlined communications to the user by presenting a session to the user which, based on at least the user profile 180, is most likely to aid the user addressing obesity in the user. This directed approach can reduce the need for multiple communications with the user, thereby reducing bandwidth and increasing the benefit of the user-computer interaction.

In some embodiments, the user profile 180 may identify or include information on a treatment regimen undertaken by the user, such as a type of treatment (e.g., therapy, pharmaceutical, or psychotherapy), duration (e.g., days, weeks, or years), and frequency (e.g., daily, weekly, quarterly, annually), among others. The user profile 180 may be stored and maintained in the database 175 using one or more files (e.g., extensible markup language (XML), comma-separated values (CSV) delimited text files, or a structured query language (SQL) file). The user profile 180 may be iteratively updated as the user provides responses, makes selections, and performs actions related to the session, the data collector 140, or the intervention generator 160. For example, the user profile 180 can be updated with data collected by the data collector 140.

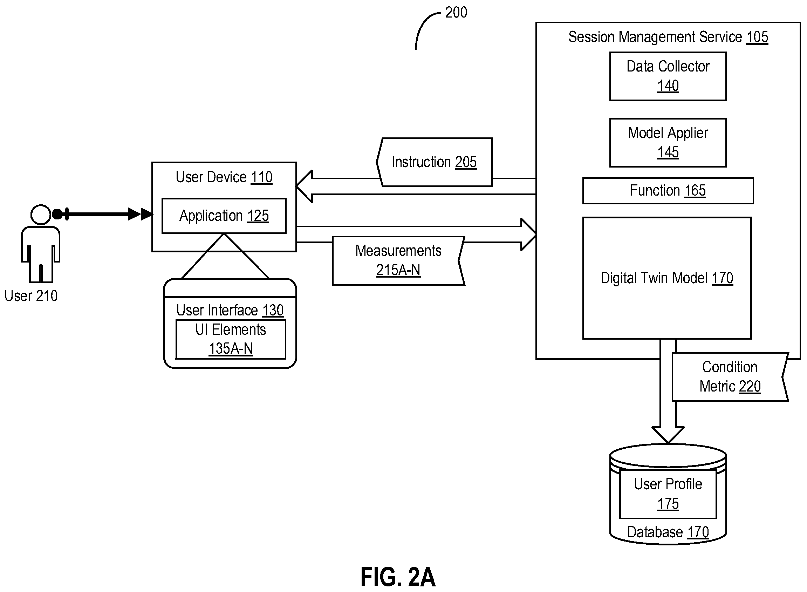

Referring now to FIGS. 2A-B, depicted are block diagrams for a process 200 to provide a session to a user 210 to generate an intervention in the system 100. The process 200 may include or correspond to operations performed by the system 100 to receive and process data provided by the user 210. Starting with FIG. 2A, under the process 200, the data collector 140 executing on the session management service 105 may create, write, or otherwise generate one or more instructions 205 (hereinafter generally referred to as instructions 205).

The instructions 205 may be to request for measurements from the user 210. The instruction 205 may identify one or more measurement fields to be acquired via the user device 110. The one or more measurement fields may be of a metabolic system of the user 210 measured over a time period. The time period may be any time period prior to sending the first instruction, and may have any length of time (e.g., 1 second, 1 day, 1 week, 1 month, 1 year). The one or more measurement fields of the metabolic system may include at least one of glucose excretion, an evaluation of an infradian or circadian rhythm, energy expenditure, physical activity, hormone levels, body weight, body fat percentage, a genetic marker, an evaluation of the gut microbiome, or energy intake, among others.

In generating the instructions 205, the data collector 140 may identify the one or more measurement fields based on at least one of the user profile 180 or past interventions stored in the database 175. For example, the user profile 180 can contain conditions the user has suffered from in the past and present which can be used to determine the intervention.

The instructions 205 may take various formats associated with the application 125. In some embodiments, the instructions 205 may be displayed, rendered, or otherwise presented via the user interface 130 of the application 125 to the user 210. The instructions 205 may take a form of a list, table, database, graph, or chart, among others displaying the one or more measurement fields for the user 210 to input. In some embodiments, the instructions 205 may include a short message service (SMS) (e.g., text message) or multimedia message service (MMS) (e.g., audio message, video message). For example, the instructions 205 may include a link to open the application 125 directing the user to input the one or more measurement fields.

Upon receipt of the first instruction, the application 125 on the user device 110 can display, render, or otherwise present the first instruction 205 via the user interface 130. The application 125 may then prompt or direct the user 210 to provide measurements 215A-N (generally referred herein after as one or more measurements 215) for each of the one or more measurement fields. The measurements 215 can be a text string, character string, or a numerical value, among others. The data collector 140 can then receive the measurements 215, and correspond each of the measurements 215 to respective measurement fields.

In various embodiments, the measurements 215 are continuously updated based on a wearable technology provided to the user. The wearable technology can obtain measurements 215 for the one or more measurement (e.g., physiological measurements). The wearable technology can include fitness trackers, smart watched, heart monitors, or pedometers, among other. The measurements 215 can also be determined by lab results or food logs provided by the user 210. In some embodiments, the measurements 215 may be obtained via the sensors of the user device 110. In some aspects, the sensors of the user device may include a wearable technology. For example, the sensors may comprise a wearable continuous glucose monitor, a smart watch capable of monitoring the user's heart rate and biometrics, a scale, or other technologies capable of obtaining data associated with the user's physiological health.

The model applier 145 can apply the measurements 215 to the function 165 of the digital twin model 170 to generate an output for a subsequent time period. The output may include a digital twin of the user 210 and visual outputs corresponding to the intervention. The subsequent time period may be a time period after the output is generated having a length of time (e.g., 1 second, 1 day, 1 week, 1 month, 1 year). The digital twin model 170 may be a phenotype-driven model which uses the energy balance equation to incorporate GE, EE, PA, and EI. GE, EE, PA, and EI can represent biological processes that influence energy balance and weight management in, for example, the user 210. The digital twin model 170 can then analyze the measurements 215 via the energy balance equation to generate hypotheses on metabolic characteristics and potential responses to different interventions of the user 210.

The function 165 for the digital twin model 170 can be a filter. The filter can include at least one of a Kalman filter, a particle filter, a sliding window filter, an information filter, an extended Kalman filter, or an unscented Kalman filter, among others. The filter can estimate system states in a presence of noise and uncertainty in, for example, the measurements 215. The filter can continuously update parameters of the digital twin model 170 based on incoming, real-time measurements 215 received from the user 210. The filter can thus allow the digital twin model 170 to adapt to a physiology and lifestyle of the user 210 to create a digital twin of the user 210. The digital twin of the user 210 can simulate unique biological processes of the user 210 related to obesity and energy balance (e.g., metabolic system) which can predict a response of the user 210 to various weight loss therapies. The filter can continuously update the digital twin via the digital twin model 170 based on modifications made to the measurements 215 by the user 210.

To initialize, the model applier 145 may set values of the set of weights in the digital twin model 170 to starting values (e.g., random or defined values). The set of weights can correspond to a metabolic system of the user 210. The digital twin model 170 may process the measurements 215 via the set of weights. Based on the measurements 215, the function 165 of the digital twin model 170 can update the set of weights. In some embodiments, the function 165 can update the set of weights in response to changes in the measurements 215 applied to the digital twin model 170. Changes in the measurements 215 can occur, for example, by a continuous stream of data provided by the wearable technology worn by the user 210. The function 165 can thus update the set of weights in real-time responsive to changes in the measurements 215. In this case, the digital twin (e.g., the output of the digital twin model 170) can also be continuously updated. The function 165 can also update the set of weights in response to any one of the following: activity level, meal timing, the time of day, circadian rhythms, and sleep or wake timing, among others. This can alter the measurements 215 which can then be used by the function 165 to update the digital twin to more accurately represent the user 210. The digital twin of the user 210 can estimate variables to model the user 210's metabolic state. For example, the digital twin can estimate variables including energy density associated with weight change, energy expenditure per unit weight change, or feedback gain in response to weight fluctuations, among others. These variables can be values calculated utilizing the measurements 215.

The metric generator 150 can then receive an updated digital twin and generate at least one condition metric 220 indicative of an obesity condition in the metabolic system of the user 210 for the subsequent time period. The condition metric 220 can represent a severity of the obesity condition of the user 210 based on the measurements 215 and the updated digital twin. The condition metric 220 can also represent a prediction of the obesity condition of the user 210 over the subsequent time period. In some embodiments, a set of condition metrics 220 is generated for a predicted response of the user 210 to a set of digital therapeutic (DTx) interventions. In some embodiments, the metabolism simulator 155 can receive another instance of the digital twin, and simulate effects of the set of DTx interventions on the digital twin. The set of DTx interventions can include neuromodulation, behavior change techniques, cognitive therapies, or gut-brain axis modulation, among others. The metabolism simulator 155 can predict an impact of each of the set of DTx interventions on an energy balance, weight loss trajectory, and other metabolic parameters. The metric generator 150 can then use results of the metabolism simulator 155 to generate the set of metrics for each of the set of DTx interventions.

In some embodiments, the digital twin model 170 can include a corresponding set of scores determined by the measurements 215. The set of scores can correspond to a measurement of an overall metabolic health of the user 210 in an obesity context. For example, the set of scores can be used as a comparison between the user 210, and an average metabolic health based on age, weight, and height of the user 210. In this case, the metric generator 150 can determine the metric based on at least one of (i) an average of the set of scores, (ii) a weighted combination of the set of scores, or (iii) a comparison with a dataset comprised of a second set of scores, among others. For example, each of the set of scores can correspond to each of the measurements 215, and the metric can be the average of the set of scores. The second set of scores could be, for example, a previous set of scores of a previous digital twin for the user 210. The second set of scores also could be another user of the application 125 with similar height, weight, or condition, among others, as the user 210. The average mentioned in the foregoing could also include a weighted average.

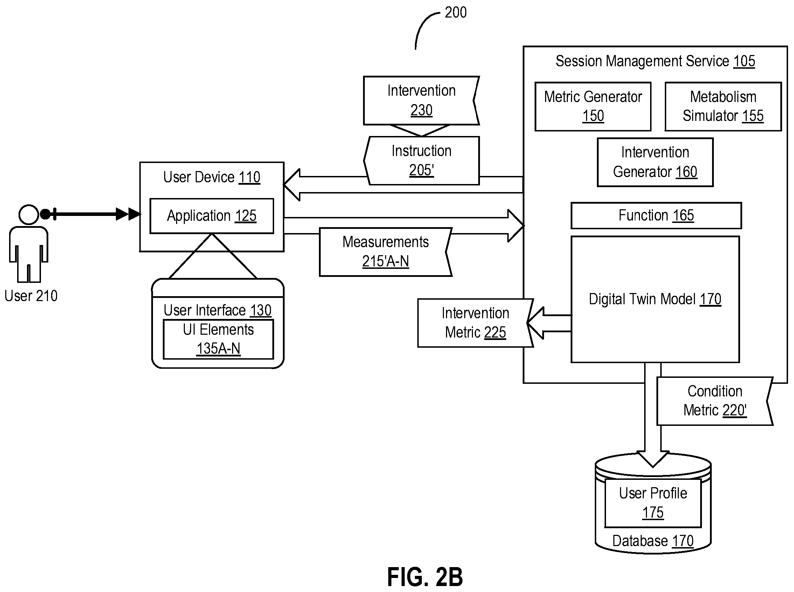

Moving onto FIG. 2B, based on the metrics, the intervention generator 160 can identify an intervention 230(e.g., treatment strategy, treatment plan, treatment, digital therapeutic) for the obesity condition of the user 210. For example, the intervention generator 160 can receive the set of metrics and corresponding set of DTx interventions (e.g., intervention 230) from the metric generator 150, and select the DTx interventions to recommend to the user 210 based on the metric. The metric can indicate a greater efficacy, greater engagement, or greater weight loss, among others compared to other metrics in the set of metrics.

In various embodiments, the intervention generator 160 identifies a set of intervention 230 to provide to the user 210 to select from. For example, the intervention generator 160 can identify three interventions 230 to provide to the user 210 to select from based on corresponding metrics of the interventions 230. Each of the interventions 230 can vary in a weight loss medication, physical activity, a reduction in energy intake, food choices, cognitive training, behavioral therapy, an optimal mealtime, an exercise routine, or a surgical intervention, among others. The weight loss medication can be selected from one or more of a GLP-1 receptor agonist, GIP receptor agonists, a lipase inhibitor, an amphetamine, an antidepressant, an opioid medication, or a melanocortin receptor agonist, among others. The GLP-receptor agonist can be selected from one or more of semaglutide, liraglutide, exenatide, and dulaglutide, or a GIP receptor agonist including tirzepatide, among others.

The intervention generator 160 can then provide the instruction 205' (e.g., the second instruction) to the user device 110 identifying the intervention 230 for the metabolic system of the user 210. The instruction 205' can include, for example, a flow chart, a diagram, a set of tasks, among others representing the intervention 230. In various embodiments, responsive to the intervention generator 160 identifying two or more interventions 230, the intervention generator 160 provides the instruction 205' to the user device 110 allowing the user 210 to select one intervention 230 of the set of the interventions 230.

In some embodiments, the metabolism simulator 155 may generate at least one intervention metric 225 to indicate a condition of the metabolic system of the user 210 in response to the identification of the intervention 230. The intervention metric 225 can be a result of the metabolism simulator 155 simulating the user 210's energy balance, weight loss trajectory, energy intake, energy output, physical activity, glucose excretion, a simulation of a body composition change, a metabolic rate, a weight loss prediction, glucose fluctuations, or energy expenditure, among others. The intervention metric 225 can represent, for example, possible progress of the user 210 in response to the administration of the intervention 230. The intervention metric 225 can communicate to the user 210 potential changes to the metabolic system of the user 210 by following the intervention 230.

In some embodiments, following identification of the intervention 230, the intervention generator 160 also can send an instruction 205' identifying the intervention 230 for the obesity condition to the user 210. Administration of the intervention 230 can result in a reduction of BMI, body fat percentage, blood glucose, or body weight, among others. In some aspects, an effective treatment or amelioration may include a reduction in 1%, 2%, 5%, 10%, 15%, 20% or more in the user's body fat composition. In some aspects, the effective treatment or amelioration may include a reduction in 1lbs, 2 lbs, 5 lbs, 10 lbs, 15 lbs, 20 lbs or more in the weight of the user. In some aspects, the effective treatment or amelioration may include a reduction in the user's cholesterol, blood glucose, or hormone levels. In some aspects, the effective treatment or amelioration may include a positive assessment of the user's psychological state, including evaluations on the quality of life.

The intervention 230 can be administered over a period of 1 day, 5 days, 1 week, 2 weeks, 1 months, 3 months, 6 months, 1 year or longer. For example, responsive to the intervention 230 including, for example, increasing sleep time, the intervention generator 160 can administer the intervention 230 by requesting the user 210 to input sleep time on a regular basis (e.g., daily). The intervention generator 160 can also send reminders to the application 125 for the user 210 to input the physical activity, and can recommend types and time lengths of physical activity for the user 210.

In some embodiments, the metabolism simulator 155 can also generate a visual output based on the simulated intervention metric 225 to the user device 110. The visual output can be an avatar of the digital twin based on a body composition change resulting from the simulated introduction of the intervention, an AI generated image, an infographic, a graphical output, or a metric of metabolic health. The visual output can show the user 210 potential results and changes to the metabolic system of the user 210 by following the proposed intervention 230. The visual output can be selected (e.g., updated) based on preferences of the user 210 which can be stored in the user profile 180. The preferences of the user 210 can include any one of metrics of metabolic health, graphical outputs, or visual format of the interface (e.g., user interface 130), among others. With the generation, the metabolism simulator 155 can send an instruction to render the visual output to the application 125 for presentation via the user interface 130.

The metabolism simulator 155 can also generate presentations of personalized weight loss projections of the digital twin, showcasing expected weight loss over time under various treatment (e.g., the intervention 230) scenarios. These projections can be adjusted based on real-time data input, allowing the user 210 to see how changes (small or large) in their behavior impact their projected outcomes. In various embodiments, responsive to the measurements 215 including chronotype data, the metabolism simulator 155 can provide tailored insights based on individual chronobiological patterns. This can include suggestions for optimal meal timing and exercise routines based on their natural sleep-wake cycle. The metabolism simulator 155 can also provide visual outputs responsive to the measurements 215 including additional data inputs such as, glucose fluctuations, among others. With the generation, the metabolism simulator 155 can send an instruction to render the visual output to the application 125 for presentation via the user interface 130.

In some embodiments, the data collector 140 can generate visual outputs to send to the user device 110. For example, the data collector 140 can generate interactive charts and graphs generated by the metabolism simulator 155 can display changes in body fat percentage and muscle mass over time based on the measurements 215. The user 210 can thus see how their body composition is expected to shift in response to different interventions 230, providing tangible evidence of progress and motivating further adherence. As another example, the data collector 140 can also display dynamic visualization of the user 210's metabolic rate throughout the day, highlighting peaks and valleys based on activity levels, meal timing, or chronotype, among others. This can help the user 210 understand when their body is most efficient at burning calories, guiding optimal meal and exercise scheduling.

In some embodiments, following administration of the intervention 230, the application 125 can send, provide, or otherwise transmit one or more measurements 215'A-N (hereinafter generally referred to as measurements 215'). The measurements 215' may be similar in form to the measurements 215 described herein. In some embodiments, the intervention 230 can be adjusted by the intervention generator 160 based on continuous updates to the measurements 215' and the digital twin model 170. For example, responsive to receiving real-time data from the wearable technology of the user 210, the intervention 230 can be adjusted to increase length of physical activity or sleep time.

The function 165 can then update the set of weights of the digital twin model 170, and the digital twin model 170 can be updated based on the information. The metric generator 150 can then generate a condition metric 220' of the metabolic system of the user 210 based on the information. For example, the information can update the measurements 215 initially provided by the user 210 which can update the digital twin. The metric generator 150 can then generate the condition metric 220' based on the digital twin to represent the metabolic system of the user 210 in response to the administration of the intervention 230.

In some embodiments, the data collector 140 can determine whether the administration of the intervention 230 resulted in (i) a decrease from the condition metric 220 by a first predetermined margin or (ii) an increase from the condition metric 220 by a second predetermined margin. The increase or decrease may correspond to a difference between the condition metric 220 prior to the intervention 230 and the condition metric 220' subsequent to the intervention 230. The condition metric 220 can be based on the set of weights of the digital twin. The decrease of the condition metric 220 can indicate efficacy (e.g., weight loss) of the intervention 230 on the user 210, while the increase of the condition metric 220 can indicate inefficacy (e.g., weight gain) of the intervention 230.

In some embodiments, the data collector 140 can also determine that the administration of the intervention 230 resulted in the condition metric 220' being (i) decreased from the condition metric 220 by a first predetermined margin or (ii) increased from the condition metric 220 by a second predetermined margin. The condition metric 220', being for a subsequent time period compared to the condition metric 220, being decreased can indicate efficacy while being increased can indicate inefficacy. Responsive to the condition metric 220 or the condition metric 220', among others, indicating inefficacy of the intervention 230, an intervention can be generated by the intervention generator 160 for the user 210. The intervention can then be administered to the user 210. The intervention can be generated based on an updated digital twin based on the condition metric 220 and the condition metric 220', or selected from the set of interventions 230 generated in response to the measurements 215.

The data twin model 170 may be updated again using additional incoming measurements from the user 210. In some embodiments, the metabolism simulator 155 can generate another intervention metric (e.g., a third metric) of the metabolic system of the user 210 based on the administration of the intervention to the user 210. In some embodiments, the metabolism simulator 155 can generate the follow up intervention metric based on the administration of the intervention. The intervention metric can represent a predicted metric of the metric generator 150 based on updated measurements 215 following administration of the intervention.

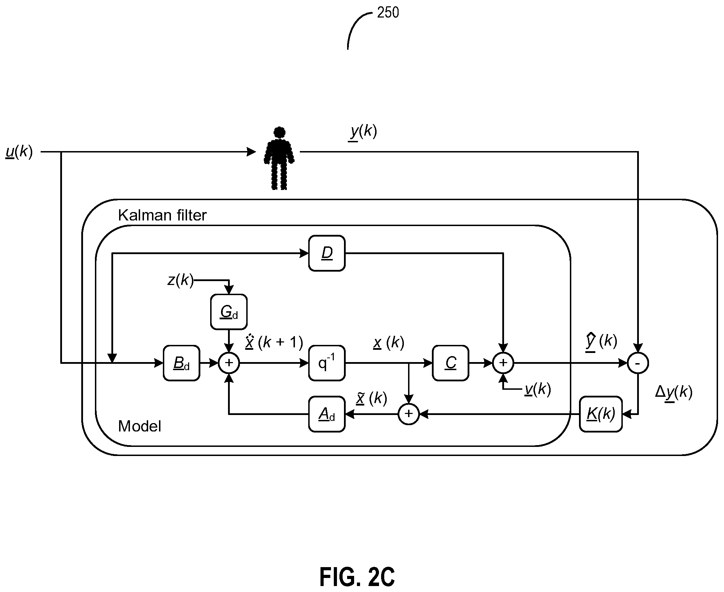

Referring now to FIG. 2C, depicted is a block diagram of an architecture 250 for the digital twin model and a Kalman filter. The architecture 250 depicted may be an example of the digital twin model 170 and the function 165. Data metabolic data from the user *y*(*k*) may be measured in response to applying therapy represented by *u*(*k*). The Kalman filter utilizes a Kalman filter function *K*(*k*) to continuously update the weights of the model based on the difference Δ*y*(*k*) between measured metabolic data from the user *y*(*k*) and the previous predicted metabolic data ŷ(*k*) from the model. The model may use the therapy *u*(*k*) and outputs from the Kalman filter *K*(*k*) as inputs, and process them using a set of parameters *A_{A}, B_{A}, C, D,* and *G_{d}.* This process may be iteratively performed as additional metabolic data is measured from the user and additional therapies are applied to the user.

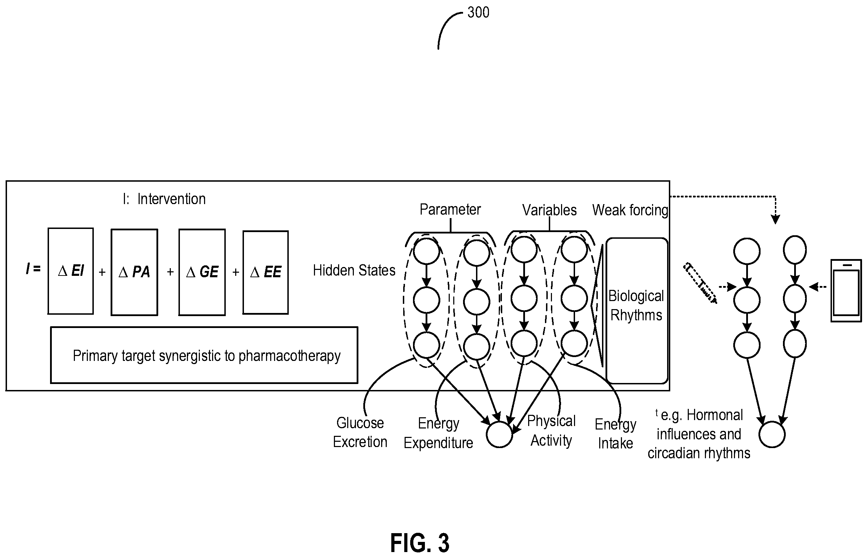

FIG. 3 depicts a process 300 used by a model (e.g., the digital twin model 170) to generate a digital twin of the user (e.g., the user 210), in accordance with an illustrative embodiment. The process 300 may include or correspond to operations performed by the system 100 to generate a digital twin. Under the process 300, the model may receive data (e.g., measurements 215) from the user and input the data into an energy balance equation. The process 300 can leverage a perturbation ansatz to simulate dynamic interplay of pharmacotherapy and digital therapeutics (e.g., the intervention 230). The process 300 can identify synergistic combinations to maximize therapeutic impact of pharmacotherapy on energy intake. The process 300 can take into account GE and EE as parameters, and PA and EI as variables as well as biological rhythms (e.g., hormonal influences and circadian rhythms) to determine change in body weight as a result of the pharmacotherapy and digital therapeutics. The digital twin can then be used to determine various digital therapeutics for the user.

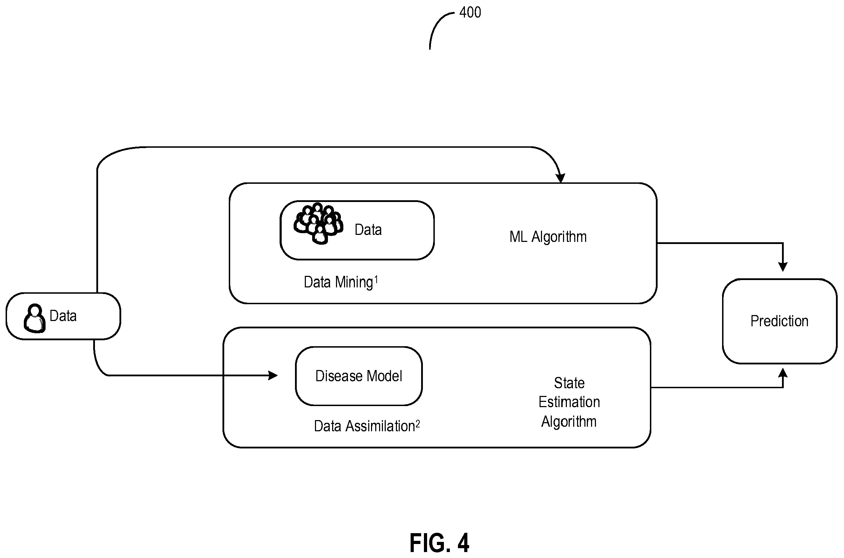

FIG. 4 depicts a block diagram for a process 400 to generate the intervention using a Kalman filter (e.g., the filter of the digital twin model 170), in accordance with an illustrative embodiment. The Kalman filter is a recursive algorithm that can be used to estimate a state of a dynamic system from a series of noisy measurements (e.g., error or variation). For example, the filter of the digital twin model 170 can estimate a state of the metabolic system of the user 210 based on the measurements 215. The Kalman filter can be based on a mathematical model of the system and noise characteristics of the measurements. Data received by the Kalman filter, under the process 400, can be input into an ML algorithm and a state estimation algorithm. The ML algorithm can perform data mining of the data to discover patterns in the data as well as to build predictive models based on the data. The state estimation algorithm can perform data assimilation of the data using a disease model to build forecasting models or state estimation algorithms. The Kalman filter can thus predict outcomes of combined pharmacotherapy and digital intervention inputs (e.g., the intervention 230). The Kalman filter can continually update the outcome based on changes in the data and evolving engagement of the user 210 with the intervention 230.

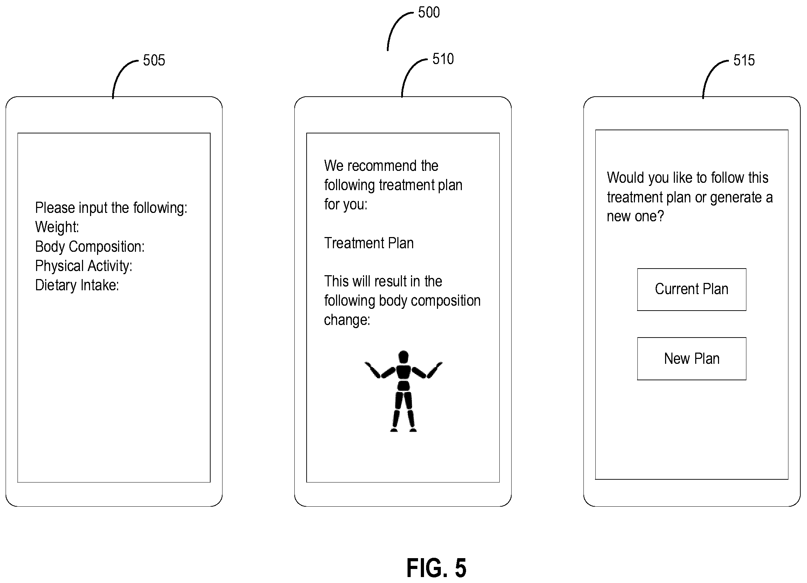

FIG. 5 depicts screenshots of a set 500 of example user interfaces for generating the intervention, in accordance with an illustrative embodiment. The user interfaces in the set 500 may be part of the application 125, and presented through the user interface 130. The user interface 505 may be a prompt to the user to input values (e.g., one or more measurements) corresponding to the measurement fields displayed. The user can then input a numerical value, text string, or character string, among others, into the measurement fields. The user interface 510 can provide a treatment plan (e.g., intervention) to the user based on the input values provided by the user. The user interface 510 can also include a visual output of body composition changes of the user as a result of following the treatment plan. The user interface 515 can provide the user an option to select a currently displayed plan as shown in the user interface 510, or select a new plan. The new plan can also be based on the input values provided by the user.

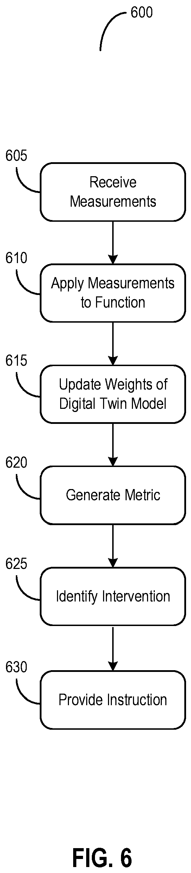

FIG. 6 depicts a flow diagram of a method 600 for identifying an intervention for the user to address obesity in accordance with an illustrative embodiment. The method 600 may be performed by any components of the system 100, such as the session management service 105, the user device 110, or the user 210, among others. Under the method 600, one or more processors can receive measurements of a user (605). The measurements can be input by the user for a measured time period. The one or more processors can then apply the measurements to a function (610). The function can be a filter. An output of the function can be for a subsequent time period of the measured time period. The one or more processors can update a set of weights of a digital twin of a user (615). The digital twin can be a machine learning model and can include the filter. The set of weights can correspond to the metabolic system of the user as well as the measurements. The one or more processors can generate a metric (620). The metric can be indicative of an obesity condition in the metabolic system of the user for the subsequent time period. The one or more processors can identify an intervention (625). The intervention can be based on the metric, and can include one or more of a weight loss medication, physical activity, a reduction in energy intake, food choices, cognitive training, behavioral therapy, an optimal mealtime, an exercise routine, and a surgical intervention. The one or more processors can provide an instruction to the user (630). The instruction can be provided by a user device and can identify the intervention for the user.

### B. Systems and Methods for Ameliorating Obesity in Users via Administration of Digital Therapeutics

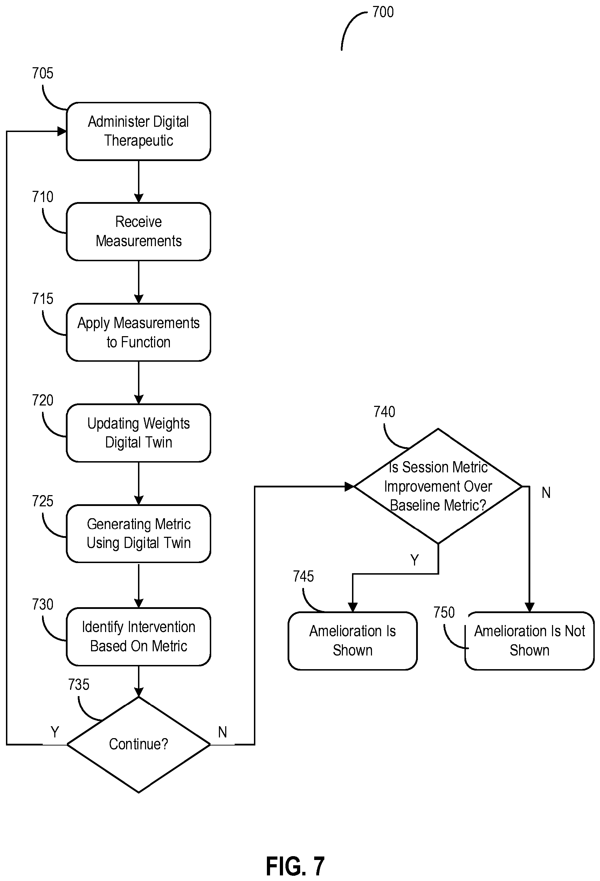

FIG. 7 depicts a flow diagram of a method 700 for ameliorating an obesity in a user. The method 700 may be performed by any components of the system 100, such as the session management service 105, the user device 110, or the user 210, among others. Under the method 700, one or more processors can administer a digital therapeutic to a user (705). The digital therapeutic can be targeted at addressing weight loss in the user. The one or more processors can receive one or more measurements of the user (710). The one or more measurements can be of a metabolic system of the user over a measured time period. The one or more processors can apply the one or more measurements to a function (715). The function can be a filter (e.g., a Kalman filter), and the filter can generate an output for a subsequent time period. The one or more processors can update, using the output of the function, at least one of a plurality of weights of a digital twin of the user (720). The plurality of weights can correspond to the metabolic system of the user. The one or more processors can continuously update (e.g., in real-time) the plurality of weights on receiving the one or more measurements. The one or more processors can generate a metric, using the digital twin, indicative of an obesity condition in the metabolic system of the user for the subsequent time period (725). For example, the metric can represent a glucose parameter. The one or more processors can then, based on the metric, identify an intervention for a metric such as a condition of the user (730). The one or more processors can generate the metric before identifying the intervention and after administration of the user. The metric prior to identifying the intervention can be a baseline metric, and after administration can be a session metric. The one or more processors can decide whether or not to continue with the digital therapeutic (735). This can be based on an efficacy of the intervention or a length of time of the intervention, among others. The one or more processors can determine whether the session metric shows an improvement over the baseline metric (740). The method 700 may include determining that an amelioration is shown when the session metric is determined to have improved over the baseline metric (745). The method 700 may include determining that no amelioration is shown when the session metric is determined to have not improved over the baseline metric (750).

In further detail, the method 700 may include administering a digital therapeutic to the user. The digital therapeutic may be associated with a user (e.g., the user 210) suffering from obesity. The obesity may be a condition, or a side effect of a disease such as metabolic disorders (e.g., diabetes, hyperthyroidism, metabolic syndrome, etc.). The obesity may be a result of lifestyle factors (e.g., socioeconomic status, genetics, unhealthy diet, etc.). The impact of obesity on the user can include physical health effects (e.g., cardiovascular diseases, diabetes, musculoskeletal problems, etc.) as well as mental health effects (e.g., depression, body image, etc.).

The user may be of any demographic or trait, such as by age (e.g., an adult (above age of 18) or late adolescent (between ages of 18-24)) or gender (e.g., male, female, or non-binary), among others. In at least partial concurrence with the time instance or the sessions during which the user is to perform interventions, the user may be on a medication to address the pathology. For example, the medication may include metformin, sulfonylureas, GLP-1 receptor agonists, DPP-4 inhibitors, SGLT2 inhibitors, insulin, thiazolidinediones, alpha-glucosidase inhibitors, orlistat, phentermine-topiramate, liraglutide, naltrexone-bupropion, semaglutide, phentermine, or setmelanotide, among others. The medication may be of an effective amount (e.g., dosage or frequency), which may be sufficient to effect positive outcomes or reduction in symptoms (e.g., reduction in weight). The efficacy of the medication in terms of addressing the user's obesity may be improved or enhanced from the concurrence with the intervention performed and monitored via the digital therapeutics application.

In some embodiments, there may be at least two groups for the trial to test the efficacy of the digital therapeutics application. The first group may include a set of users (e.g., including the user) for a treatment group. The users of the first group may be exposed to or provided with the intervention to be performed by the user via the digital therapeutics application (e.g., the application 125 or the Study App described herein). As discussed herein, the intervention may include, for example, physical activity, surgical intervention, or diet changes, or any combination thereof. The second group may include another set of users for a control group. The users of the second group may be exposed to or provided with a control therapy (sometimes herein referred to as a placebo or standard treatment).

The control therapy may exclude the tasks aimed at addressing the obesity and to be performed via the digital therapeutics application. In some embodiments, the control therapy may include an intervention performed via an application on a computing device that is inert with respect to the obesity or related symptoms. The task may include any intervention besides the physical activity, surgical intervention, or diet changes as described herein for the digital therapeutic application. In some embodiments, the control therapy may include an inert version of the intervention without any association to obesity. The inert version of the intervention may lack proper generation of the intervention per user. The application for the control therapy may also lack, physical activity, surgical intervention, and diet changes.

In some embodiments, the control therapy may include an inert version of physical activity in which users are presented with prompts to perform intervention tasks unrelated to the obesity. For instance, the inert version of the physical activity may prompt users to walk for 5 minutes per day, without any association to a metabolic system of the user. In some embodiments, the control therapy may include an inert version of surgical intervention. For example, the inert version of surgical intervention may not include surgical intervention. The control therapy may include other forms of cognitive behavioral therapy.

In some embodiments, the control therapy may include a care-as-usual (CAU) treatment regimen. The CAU treatment regimen may lack the digital therapeutics application described herein. For example, the CAU treatment regimen may have the user receive medication for the obesity on a scheduled basis (e.g., one to three times a day), and may include assessments of the obesity conducted in person or via a web application to evaluate symptoms. Baseline metrics (e.g., the metric before the intervention) may be obtained (e.g., via a computing device) from users of both the treatment group and the control group (e.g., receiving an inert version or CAU).

The method 700 may include receiving one or more measurements of the user (710). The one or more measurements may be related to a metabolic system of the user and include at least one of glucose excretion, an evaluation of an infradian or circadian rhythm, energy expenditure, physical activity, hormone levels, body weight, body fat percentage, a genetic marker, an evaluation of the gut microbiome, or energy intake, among others. The one or more measurements may be input by the user into the digital therapeutics application or received from various data sources. The various data sources can include blood tests, logs, or wearable technology, among others. The wearable technology can be, for example, a glucose monitor worn by the user.

The method 700 may include applying the one or more measurements to a function (715). The function can be a filter, and specifically a Kalman filter. The Kalman filter can be included in a digital twin model.

The method 700 can include updating a plurality of weights of the digital twin model of the user (720). The Kalman filter can constantly update the plurality of weights of the user as the one or more measurements are receives (e.g., in real-time). The digital twin model can thus output a digital twin of the user. The digital twin model can be a phenotype-driven model and utilize the energy balance equation to output the digital twin of the user.

The digital twin model can simulate various interventions on the digital twin to select the intervention. The method 700 may include generating a metric using the digital twin (725). The metric can be a first metric (e.g., baseline metric, baseline measure) calculated prior to the user following the intervention. The metric can also be a second metric (e.g., session metric) calculated after the user begins following the intervention.

The baseline measure may be (e.g., by a computing device) obtained prior to performing any tasks of the intervention is to be performed by the user via a digital therapeutic application (e.g., the application 125 or the Study App described herein). The baseline measure may indicate the condition of the user with respect to the severity of the obesity. The baseline metric may include, for example, a weight, a weight efficacy lifestyle questionnaire (WEL) value, a body weight index (BMI) value, or computer assessment value for inhibitory control, among others. Before performance of the intervention, the user may have an initial baseline metric satisfying a baseline threshold. The threshold may delineate, define, or identify a value for the initial metric at which the user is identified to be suitable or eligible for performance of the tasks via the digital therapeutic application. The initial metric may be, for example, the BMI and the baseline threshold may be a value of 30 or less.

The session metric may be associated with the user (e.g., the user 210) with respect to obesity. The session metric may be obtained (e.g., by a computing device) subsequent to performing at least one of the time instances or sessions during which a task is to be performed by the user via the digital therapeutic application. The baseline measure may indicate the condition of the user with respect to the severity of the obesity and the associated impact on the user's daily life and activities, as a result of the performance of the intervention. The session metric may include, for example, a weight, a weight efficacy lifestyle questionnaire (WEL) value, a body weight index (BMI) value, or computer assessment value for inhibitory control, among others. The session metric may be of the same type of measurement as the baseline metric. The session metrics may be obtained from users of both the treatment group and the control group.

The method 700 may include identifying an intervention based on the metric (730). The intervention may be to lessen, alleviate, any symptoms of the obesity, or reduce a weight of the user by having the user perform the intervention. The intervention may include at least one of a weight loss medication, physical activity, a reduction in energy intake, food choices, cognitive training, behavioral therapy, an optimal mealtime, an exercise routine, and a surgical intervention. The intervention may be selected based on the digital twin and the metric generated by the digital twin.

The method 700 may include identifying or determining whether to continue (735). The determination may be based on the set length (e.g., days, weeks, or years) of the trial, a set number of time instances during which to perform one or more tasks, or a set number of tasks to be provided to the user. For example, the set number of time instances may range between 2 weeks to 6 months, relative to the obtaining of the baseline metric. When the amount of time from the obtaining of the baseline metric exceeds the set length, the determination may be to stop providing additional tasks.

In contrast, when an amount of time from the obtaining of the baseline metric has not exceeded the set length, the determination may be to continue providing additional interventions and repeat from (710). The selection or modification of the intervention for the next time instance or session may be based on the performance of the task in the previous time instance or session. In some embodiments, the next intervention may be modified based on response data from the user while performing the task in the prior time instance or session. In some embodiments, the next intervention may be modified based on a score determined from the performance of the task in the prior time instance or session. In some embodiments, the next intervention may be modified based on updates to the plurality of weights of the digital twin model based on the one or more measurements. The intervention may be modified, for example, by order of performance (e.g., with respect to other intervention), a time length to complete the intervention, and a difficulty of performing the intervention, among others. The selection or modification of intervention for the next time instance or session may be independent from the performance of the intervention in the previous time instance or session.

The method 700 may include identifying or determining whether the session metric is an improvement over the baseline metric (740). The improvement may correspond to the reduction in weight or the intensity of the side effects related to obesity. The improvement may be shown when the session metric is increased compared to the baseline metric by a first predetermined margin or when the session metric is decreased compared to the baseline metric by a second predetermined margin. The margin may identify or define a difference in value between the baseline and session metrics at which to determine that the user shows reduction in in weight or the intensity of the side effects related to obesity. Whether the improvement is shown by increase or decrease may depend on the type of metric used to measure the user with respect to the obesity. The margin may also depend on the type of metric used, and may in general correspond to the difference in value showing noticeable difference by the clinician or user with respect to the obesity, or showing a statistically significant result in the difference in the values between the baseline and session metrics.

In some embodiments, at least one of the first predetermined margin or the second predetermined margin may be calculated, generated, or otherwise determined (e.g., by a computing device). The first predetermined margin or the second predetermined margin may be based on the baseline and session metrics obtained from the treatment group or the control group of users. The margin may correspond to a difference between a change in baseline and session metrics in the treatment group versus a change in baseline and session metrics in the control group (e.g., receiving the control therapy) that is statistically significant. The difference may be based on any number of statistical tests, such as a t-test, a z-test, chi-squared test, among others.

The method 700 may include determining that amelioration is shown when the session metric is determined to be an improvement over the baseline metric (745). In some embodiments, the amelioration may be determined (e.g., by the computing system or a clinician examining the user) to occur when the session BMI metric is decreased from the baseline BMI metric by the first predetermined margin. In some embodiments, the amelioration may be determined to occur when the session WRI metric is decreased from the baseline WRI metric by the second predetermined margin. In some embodiments, the amelioration may be determined to occur when the session Diabetes Risk Score metric is decreased from the baseline Diabetes Risk Score metric by the second predetermined margin. In some embodiments, the amelioration may be determined to occur when the session body fat percentage metric is decreased from the baseline body fat percentage metric by the first predetermined margin.

The method 700 may include determining that amelioration is not shown when the session metric is determined to be not an improvement over the baseline metric (750). In some embodiments, the amelioration may be determined (e.g., by the computing system or a clinician examining the user) to not occur when the session BMI metric is not decreased from the baseline BMI metric by the first predetermined margin. In some embodiments, the amelioration may be determined to occur when the session WRI metric is not decreased from the baseline WRI metric by the second predetermined margin. In some embodiments, the amelioration may be determined to occur when the session Diabetes Risk Score metric is not decreased from the baseline Diabetes Risk Score metric by the second predetermined margin. In some embodiments, the amelioration may be determined to occur when the session body fat percentage metric is not decreased from the baseline body fat percentage metric by the first predetermined margin.

### Example 1: Efficacy of Digital Twin in a Digital Therapeutics to Target Obesity

In one example, the CT-100 mobile app (e.g., the application 125) is given to individuals with a self-reported weight based on a weight efficacy lifestyle questionnaire (WEL). WEL is a self-report instrument to assess an individual's control of eating behaviors in various situations and can help determine which areas the individual may need more support in. The treatment is about 2-30 weeks long. This study is a single-center, exploratory, multiple-arm study to evaluate the overall effects of an abbreviated version of CT-100 as a treatment for patients 18 and older with a BMI of greater than or equal to 30 kg/m². There are at least 20 participants per arm.

Participants who meet eligibility criteria are enrolled in the study and are randomized to one of the study arms at the Screening Visit. Participants in different arms of the study use different Study Apps as well as a Digital Control. The Study App includes different versions of the CT-100 mobile app and the Digital Control is a control mobile app. The difference in study arms is the different versions of CT-100 and/or different drugs the participants were taking.

Screening Period: All participants are screened for up to 14 days, including electronic informed consent. Assessments during this period are performed remotely according to the Schedule of Activities and Assessments (SoA). Participants are considered eligible if they meet all inclusion criteria and no exclusion criteria, based on investigator assessment. Screening and a Baseline Visit can occur on the same day.

Baseline Visit: Participants are contacted for a Baseline Visit to review and confirm eligibility and perform identity verification. Assessments are performed according to the SoA. Eligible participants are randomized during a virtual study visit on Day 1. Participants are randomized 1:1 (Study App:Digital Control). Participants randomized download and activated their respective app onto their personal primary iPhone or Android smartphone.

Intervention Period: Study App group participants utilize an app for a certain number of minutes per day for 2-30 weeks. Control group participants receive a Digital Control App. Participants assigned to the Study App group complete the user experience questionnaire at the end of the Intervention Period

Early Follow-up Period: Participants enter an up to 1-week follow-up period in which they attended a visit to complete follow-up assessments. Participants complete follow-up assessments according to the SoA. Participants do not perform any activities within their respective app, which will be deactivated and uninstalled. A subset of participants from the Study App intervention arm completed an optional qualitative before, during, or after the follow-up period.

Mid-Term Follow-up Period: Participants entered an up to 1-week follow-up period about 2-30 weeks after the Intervention Period. Participants complete follow-up assessments according to the SoA.

Long-Term Follow-up Period: Participants entered an up to 1-week follow-up period 30-52 weeks after the Intervention Period. Participants complete follow-up assessments according to the SoA.

Inclusion Criteria: A participant eligible for entry into the study if all of the following criteria are met, including:
1. Age 18+;
2. Fluent in written and spoken English (confirmed by ability to read and comprehend the informed consent form);
3. Lives in the United States;
4. Has an active email address;
5. Willing and able to receive email messages;
6. Has access to internet connection during the study duration;
7. Has an active payment account to receive study compensation, or is willing to create one;
8. Willing and able to comply with study protocol and assessments;
9. Is the solo user of an iPhone with iPhone operating systems (iOS) 10 or greater capabilities or a smartphone with an Android operating system (OS) 5 or greater capabilities with cellular and/or internet access for the duration of the study period;
10. Is willing and able to receive Short Message Service (SMS) text messages and notifications on their smartphone; and
11. BMI ≥ 30 kg/m², as calculated by self-reported height and weight.

Exclusion Criteria: A participant is eligible for study entry if any of the following criteria are met:
1. Pregnant or planning to become pregnant;
2. Visual, dexterity or cognitive deficit so severe that it precludes the use of an app-based activity per investigator judgment;
3. Severe psychiatric disorder involving a history of psychosis (e.g. schizophrenia, bipolar disorder, severe personality disorders);
4. Severe neurological disorders impairing brain function (e.g., previous stroke, dementia, primary brain tumors, brain metastases, Alzheimer's disease, Parkinson's disease, history of significant head trauma followed by persistent neurologic deficits, or known structural brain abnormalities.);
5. Self-reported substance use disorder within the past 1 year;
6. Psychiatric hospitalization in the past 6 months;
7. Participated in any research study (including studies on psychotherapy, mindfulness, cognitive training, or drug treatment) within the past 3 months;
8. Initiation or change in primary disease specific medication for 30 days prior to entering the study;
9. Planning the introduction of new therapies (including psychotherapy, mindfulness, cognitive training, or pharmacological treatment) during the study duration (2-30 weeks);
10. Anticipating a change in current pharmacological or psychotherapy treatment regimen during the study period (15 weeks);
11. A history of prior surgery for weight loss or plans to do so during the study;
12. A history of an eating disorder as defined by the Diagnostic and Statistical Manual of Mental Disorders, Fifth Edition (DSM-5);
13. A recent history (within the past 4 weeks) of taking weight loss medications, or medications or supplements known to be associated with significant weight loss or weight gain, or plans to start taking such medication/supplements during the study;
14. Current participation in any other weight loss or weight management program, or plans to do so during the study;
15. Participants with diagnosed diabetes mellitus (type 1 or type 2) or uncontrolled hypertension, history of ischemic heart disease, stroke, neurological disease; or
16. Participants who have been involved in a weight loss intervention program (including anti-obesity medication) within the past 3 months (and or loss >10% of body weight) or who have ever had bariatric surgery or have weight loss devices implanted.
17. Taking other GLP-1 receptor agonists (currently or in the past 3 months).

Exploratory Endpoints:
- Change in weight from baseline to week 47 for each group (GLP-1 RA + app versus GLP-1 RA alone).
- Change in weight from baseline to week 47 for the Study App group compared to change in weight from baseline to week 47 in the control group.
- Change in WEL from baseline to week 47 for each group (GLP-1 RA + app versus GLP-1 RA alone).
- Change in WEL from baseline to week 47 for the Study App group compared to change in WEL from baseline to week 47 in the control group.
- Change in computerized cognitive assessment measures for inhibitory control from baseline to week 47 for each group (GLP-1 RA + app versus GLP-1 RA alone).
- Change in computerized cognitive assessment measures for inhibitory control from baseline to week 47 for the Study App group compared to change in computerized cognitive assessment measures for inhibitory control from baseline to week 47 in the control group.
- The Study App will be able to establish a degree of engagement with the study participants as assessed by predefined Study App engagement metrics.
- A statistical analysis plan (SAP) will be finalized prior to database lock and will include a more technical and detailed description of the statistical analyses described in this protocol.

### Schedule of Activities and Assessments:

| **Study Day** | **Screening Period** | **Study Period** | | **Follow-Up Period** | | |
|---|---|---|---|---|---|---|
| | **Screening** | **Baseline** | **Engagement Period** | **Week 13** | **Week 23** | **Week 47** |
| | **Day -14 to Day -1** | **Day 0** | **Day 1 to Day 84** | **Day 85 to Day 91** | **Day 161 to Day 168** | **Day 329 to Day 336** |
| **Study Visit Number** | **1** | **2** | | **3** | **4** | **5** |
| **Remote Visit** | **X** | **X** | | | | |
| Informed Consent | X | | | | | |
| Demographics | X | | | | | |
| Inclusion/Exclusions Criteria | X | X | | | | |
| Download & Activate App | | X | | | | |
| PHQ-8 | | X | | | | |
| Study App Engagement | | X | X | | | |
| Self-reported weight | | X | | X | X | X |
| Weight Efficacy Lifestyle Questionnaire | | X | | X | X | X |
| Computerized performance assessment | | X | | X | X | X |
| User treatment experience (quant survey) | | | | X | | |
| Deactivate App | | | | X | | |
| AE/SAE Review | X | X | X | | | |

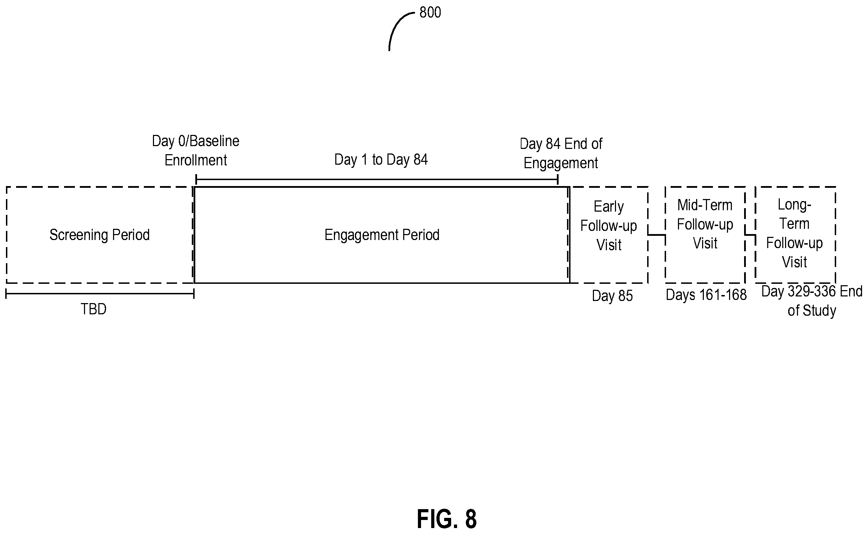

A study performed using the CT-100 mobile app in adults (18+ years) with obesity can follow a study schedule 800 as shown in FIG. 8.

### Example 2: Engagement of Digital Twin in a Digital Therapeutics to Target Obesity

In one example, the CT-100 mobile app (e.g., the application 125) is given to individuals with a self-reported weight and height. The treatment is about 2-30 weeks long. This study is a single-center, exploratory, multiple-arm study to evaluate the overall effects of an abbreviated version of CT-100 as a treatment for patients 18 and older with a BMI of greater than or equal to 30 kg/m². There are 50 participants per arm.

Participants who meet eligibility criteria are enrolled in the study and are randomized to one of the study arms at the Screening Visit. Participants in different arms of the study use different Study Apps. The patients in different arms may also use a Digital Control or may proceed without utilizing a digital control. The Study App includes different versions of the CT-100 mobile app and the Digital Control is a control mobile app. The difference in study arms is the different versions of CT-100 and/or different drugs (e.g., liraglutide, semaglutide) the participants were taking.

Screening Period: All participants are screened for up to 14 days, including electronic informed consent. Assessments during this period are performed remotely according to the Schedule of Activities and Assessments (SoA). Participants are considered eligible if they meet all inclusion criteria and no exclusion criteria, based on investigator assessment. Screening and a Baseline Visit can occur on the same day.

Baseline Visit: Participants are contacted for a Baseline Visit to review and confirm eligibility and perform identity verification. Assessments are performed according to the SoA. Eligible participants are randomized during a virtual study visit on Day 1. Participants are randomized 1:1 (Study App:Digital Control). Participants randomized download and activated their respective app onto their personal primary iPhone or Android smartphone.

Intervention Period: Study App group participants utilize an app for a certain number of minutes per day for 2-30 weeks. Control group participants receive a Digital Control App. Participants assigned to the Study App group complete the user experience questionnaire at the end of the Intervention Period.

Follow-up Period: Participants complete follow-up assessments according to the SoA. Participants do not perform any activities within their respective app. A subset of participants from the Study App intervention arm completes an optional qualitative interview before, during, or after the follow-up period.

Inclusion Criteria: A participant eligible for entry into the study if all of the following criteria are met, including:
1. Age 18+;
2. Fluent in written and spoken English (confirmed by ability to read and comprehend the informed consent form);
3. Lives in the United States;
4. Has an active email address;
5. Willing and able to receive email messages;
6. Has access to internet connection during the study duration;
7. Has an active PayPal account to receive study compensation, or is willing to create one;
8. Willing and able to comply with study protocol and assessments;
9. Is the solo user of an iPhone with iPhone operating systems (iOS) 10 or greater capabilities or a smartphone with an Android operating system (OS) 5 or greater capabilities with cellular and/or internet access for the duration of the study period;
10. Is willing and able to receive Short Message Service (SMS) text messages and notifications on their smartphone; and
11. BMI ≥ 30 kg/m², as calculated by self-reported height and weight.

Exclusion Criteria: A participant is eligible for study entry if any of the following criteria are met:
18. Pregnant or planning to become pregnant;
19. Visual, dexterity or cognitive deficit so severe that it precludes the use of an app-based activity per investigator judgment;
20. Severe psychiatric disorder involving a history of psychosis (e.g. schizophrenia, bipolar disorder, severe personality disorders);
21. Severe neurological disorders impairing brain function (e.g., previous stroke, dementia, primary brain tumors, brain metastases, Alzheimer's disease, Parkinson's disease, history of significant head trauma followed by persistent neurologic deficits, or known structural brain abnormalities);
22. Self-reported substance use disorder within the past 1 year;
23. Psychiatric hospitalization in the past 6 months;
24. Participated in any research study (including studies on psychotherapy, mindfulness, cognitive training, or drug treatment) within the past 3 months;
25. Initiation or change in primary disease specific medication for 30 days prior to entering the study;
26. Planning the introduction of new therapies (including psychotherapy, mindfulness, cognitive training, or pharmacological treatment) during the study duration (15 weeks);
27. Anticipating a change in current pharmacological or psychotherapy treatment regimen during the study period (15 weeks);
28. A history of prior surgery for weight loss or plans to do so during the study;
29. A history of an eating disorder as defined by the Diagnostic and Statistical Manual of Mental Disorders, Fifth Edition (DSM-5);
30. A recent history (within the past 4 weeks) of taking weight loss medications, or medications or supplements known to be associated with significant weight loss or weight gain, or plans to start taking such medication/supplements during the study;
31. Current participation in any other weight loss or weight management program, or plans to do so during the study;
32. Participants with diagnosed diabetes mellitus (type 1 or type 2) or uncontrolled hypertension, history of ischemic heart disease, stroke, neurological disease;
33. Participants who have been involved in a weight loss intervention program (including anti-obesity medication) within the past 3 months (and or loss >10% of body weight) or who have ever had bariatric surgery or have weight loss devices implanted; or
34. Taking other GLP-1 receptor agonists (currently or in the past 3 months).

Exploratory Endpoints:
- The Study App will be able to establish a degree of engagement with the study participants as assessed by predefined Study App engagement metrics.
- A statistical analysis plan (SAP) will be finalized prior to database lock and will include a more technical and detailed description of the statistical analyses described in this protocol.

### Schedule of Activities and Assessments:

| **Study Day** | **Screening Period** | **Study Period** | | **Follow-Up Period** |
|---|---|---|---|---|
| | **Screening** | **Baseline** | **Engagem ent Period** | **Week 13** |
| | **Day -14 to Day -1** | **Day 0** | **Day 1 to Day 84** | **Day 85 to Day 91** |
| **Study Visit Number** | **1** | **2** | | **3** |
| **Remote Visit** | **X** | **X** | | |
| Informed Consent | X | | | |
| Demographics | X | | | |
| Inclusion/Excl usions Criteria | X | X | | |
| Download & Activate App | | X | | |
| PHQ-8 | | X | | |
| Study App Engagement | | X | X | |
| Computerized performance assessment | | X | | X |
| User treatment experience (quant survey) | | | | X |
| Deactivate App | | | | X |
| AE/SAE Review | X | X | X | |

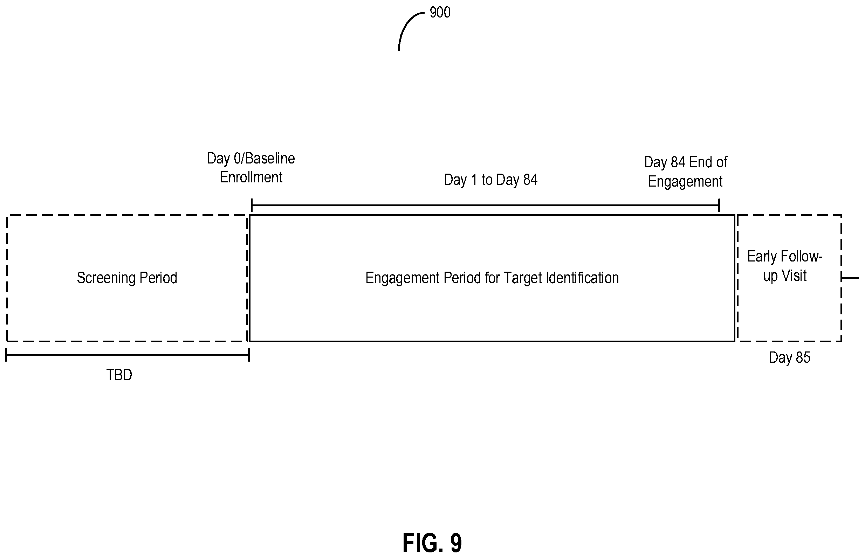

A study performed using the CT-100 mobile app in adults (18+ years) with obesity can follow a study schedule 900 as shown in FIG. 9.

Following the trial period, the users will experience an effective amelioration in obesity as measured by a reduction in body weight, body fat composition and/or percentage, blood glucose levels, and/or other psychological or physiological improvements.

### C. Network and Computing Environment

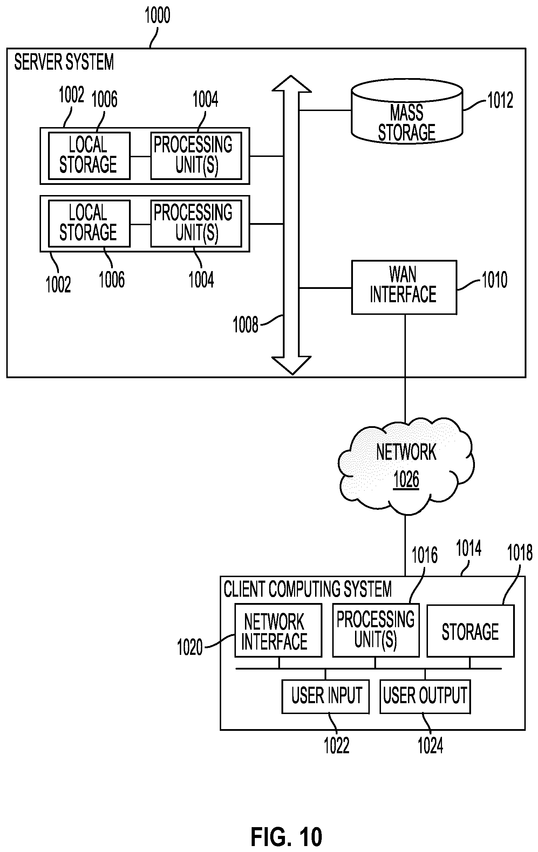

Various operations described herein can be implemented on computer systems. FIG. 10 shows a simplified block diagram of a representative server system 1000, client computing system 1014, and network 1026 usable to implement certain embodiments of the present disclosure. In various embodiments, server system 1000 or similar systems can implement services or servers described herein or portions thereof. Client computing system 1014 or similar systems can implement clients described herein. The system 100 described herein can be similar to the server system 1000. Server system 1000 can have a modular design that incorporates a number of modules 1002 (e.g., blades in a blade server embodiment); while two modules 1002 are shown, any number can be provided. Each module 1002 can include processing unit(s) 1004 and local storage 1006.

Processing unit(s) 1004 can include a single processor, which can have one or more cores, or multiple processors. In some embodiments, processing unit(s) 1004 can include a general-purpose primary processor as well as one or more special-purpose coprocessors such as graphics processors, digital signal processors, or the like. In some embodiments, some or all processing unit(s) 1004 can be implemented using customized circuits, such as application specific integrated circuits (ASICs) or field programmable gate arrays (FPGAs). In some embodiments, such integrated circuits execute instructions that are stored on the circuit itself. In other embodiments, processing unit(s) 1004 can execute instructions stored in local storage 1006. Any type of processors in any combination can be included in processing unit(s) 1004.

Local storage 1006 can include volatile storage media (e.g., DRAM, SRAM, SDRAM, or the like) or non-volatile storage media (e.g., magnetic or optical disk, flash memory, or the like). Storage media incorporated in local storage 1006 can be fixed, removable, or upgradeable as desired. Local storage 1006 can be physically or logically divided into various subunits such as a system memory, a read-only memory (ROM), and a permanent storage device. The system memory can be a read-and-write memory device or a volatile read-and-write memory, such as dynamic random-access memory. The system memory can store some or all of the instructions and data that processing unit(s) 1004 need at runtime. The ROM can store static data and instructions that are needed by processing unit(s) 1004. The permanent storage device can be a non-volatile read-and-write memory device that can store instructions and data even when module 1002 is powered down. The term "storage medium" as used herein includes any medium in which data can be stored indefinitely (subject to overwriting, electrical disturbance, power loss, or the like) and does not include carrier waves and transitory electronic signals propagating wirelessly or over wired connections.

In some embodiments, local storage 1006 can store one or more software programs to be executed by processing unit(s) 1004, such as an operating system or programs implementing various server functions such as functions of the system 100 or any other system described herein, or any other server(s) associated with system 100 or any other system described herein.

"Software" refers generally to sequences of instructions that, when executed by processing unit(s) 1004, cause server system 1000 (or portions thereof) to perform various operations, thus defining one or more specific machine embodiments that execute and perform the operations of the software programs. The instructions can be stored as firmware residing in read-only memory or program code stored in non-volatile storage media that can be read into volatile working memory for execution by processing unit(s) 1004. Software can be implemented as a single program or a collection of separate programs or program modules that interact as desired. From local storage 1006 (or non-local storage described below), processing unit(s) 1004 can retrieve program instructions to execute and data to process in order to execute various operations described above.

In some server systems 1000, multiple modules 1002 can be interconnected via a bus or other interconnect 1008, forming a local area network that supports communication between modules 1002 and other components of server system 1000. Interconnect 1008 can be implemented using various technologies, including server racks, hubs, routers, etc.

A wide area network (WAN) interface 1010 can provide data communication capability between the local area network (e.g., through the interconnect 1008) and the network 1026, such as the Internet. Other technologies can be used to communicatively couple the server system with the network 1026, including wired (e.g., Ethernet, IEEE 802.3 standards) or wireless technologies (e.g., Wi-Fi, IEEE 802.11 standards).

In some embodiments, local storage 1006 is intended to provide working memory for processing unit(s) 1004, providing fast access to programs or data to be processed while reducing traffic on interconnect 1008. Storage for larger quantities of data can be provided on the local area network by one or more mass storage subsystems 1012 that can be connected to interconnect 1008. Mass storage subsystem 1012 can be based on magnetic, optical, semiconductor, or other data storage media. Direct attached storage, storage area networks, network-attached storage, and the like can be used. Any data stores or other collections of data described herein as being produced, consumed, or maintained by a service or server can be stored in mass storage subsystem 1012. In some embodiments, additional data storage resources may be accessible via WAN interface 1010 (potentially with increased latency).

Server system 1000 can operate in response to requests received via WAN interface 1010. For example, one of modules 1002 can implement a supervisory function and assign discrete tasks to other modules 1002 in response to received requests. Work allocation techniques can be used. As requests are processed, results can be returned to the requester via WAN interface 1010. Such operation can generally be automated. Further, in some embodiments, WAN interface 1010 can connect multiple server systems 1000 to each other, providing scalable systems capable of managing high volumes of activity. Other techniques for managing server systems and server farms (collections of server systems that cooperate) can be used, including dynamic resource allocation and reallocation.

Server system 1000 can interact with various user-owned or user-operated devices via a wide-area network such as the Internet. An example of a user-operated device is shown in FIG. 10 as client computing system 1014. Client computing system 1014 can be implemented, for example, as a consumer device such as a smartphone, other mobile phone, tablet computer, wearable computing device (e.g., smart watch, eyeglasses), desktop computer, laptop computer, and so on.

For example, client computing system 1014 can communicate via WAN interface 1010. Client computing system 1014 can include computer components such as processing unit(s) 1016, storage device 1018, network interface 1020, user input device 1022, and user output device 1024. Client computing system 1014 can be a computing device implemented in a variety of form factors, such as a desktop computer, laptop computer, tablet computer, smartphone, other mobile computing device, wearable computing device, or the like.

Processing unit 1016 and storage device 1018 can be similar to processing unit(s) 1004 and local storage 1006 described above. Suitable devices can be selected based on the demands to be placed on client computing system 1014. For example, client computing system 1014 can be implemented as a "thin" client with limited processing capability or as a high-powered computing device. Client computing system 1014 can be provisioned with program code executable by processing unit(s) 1016 to enable various interactions with server system 1000.

Network interface 1020 can provide a connection to the network 1026, such as a wide area network (e.g., the Internet) to which WAN interface 1010 of server system 1000 is also connected. In various embodiments, network interface 1020 can include a wired interface (e.g., Ethernet) or a wireless interface implementing various RF data communication standards such as Wi-Fi, Bluetooth, or cellular data network standards (e.g., 3G, 4G, LTE, etc.).

User input device 1022 can include any device (or devices) via which a user can provide signals to client computing system 1014; client computing system 1014 can interpret the signals as indicative of particular user requests or information. In various embodiments, user input device 1022 can include any or all of a keyboard, touch pad, touch screen, mouse or other pointing device, scroll wheel, click wheel, dial, button, switch, keypad, microphone, and so on.

User output device 1024 can include any device via which client computing system 1014 can provide information to a user. For example, user output device 1024 can include display-to-display images generated by or delivered to client computing system 1014. The display can incorporate various image generation technologies, e.g., a liquid crystal display (LCD), light-emitting diode (LED) display including organic light-emitting diodes (OLED), projection system, cathode ray tube (CRT), or the like, together with supporting electronics (e.g., digital-to-analog or analog-to-digital converters, signal processors, or the like). Some embodiments can include a device such as a touchscreen that function as both input and output device. In some embodiments, other user output devices 1024 can be provided in addition to or instead of a display. Examples include indicator lights, speakers, tactile "display" devices, printers, and so on.

Some embodiments include electronic components, such as microprocessors, storage, and memory that store computer program instructions in a computer readable storage medium. Many of the features described in this specification can be implemented as processes that are specified as a set of program instructions encoded on a computer readable storage medium. When these program instructions are executed by one or more processing units, they cause the processing unit(s) to perform various operations indicated in the program instructions. Examples of program instructions or computer code include machine code, such as is produced by a compiler, and files including higher-level code that are executed by a computer, an electronic component, or a microprocessor using an interpreter. Through suitable programming, processing unit(s) 1004 and 1016 can provide various functionality for server system 1000 and client computing system 1014, including any of the functionality described herein as being performed by a server or client, or other functionality.

It will be appreciated that server system 1000 and client computing system 1014 are illustrative and that variations and modifications are possible. Computer systems used in connection with embodiments of the present disclosure can have other capabilities not specifically described here. Further, while server system 1000 and client computing system 1014 are described with reference to particular blocks, it is to be understood that these blocks are defined for convenience of description and are not intended to imply a particular physical arrangement of component parts. For instance, different blocks can be but need not be located in the same facility, in the same server rack, or on the same motherboard. Further, the blocks need not correspond to physically distinct components. Blocks can be configured to perform various operations, e.g., by programming a processor or providing appropriate control circuitry, and various blocks might or might not be reconfigurable depending on how the initial configuration is obtained. Embodiments of the present disclosure can be realized in a variety of apparatus including electronic devices implemented using any combination of circuitry and software.

While the disclosure has been described with respect to specific embodiments, one skilled in the art will recognize that numerous modifications are possible. Embodiments of the disclosure can be realized using a variety of computer systems and communication technologies, including but not limited to specific examples described herein. Embodiments of the present disclosure can be realized using any combination of dedicated components or programmable processors or other programmable devices. The various processes described herein can be implemented on the same processor or different processors in any combination. Where components are described as being configured to perform certain operations, such configuration can be accomplished, e.g., by designing electronic circuits to perform the operation, by programming programmable electronic circuits (such as microprocessors) to perform the operation, or any combination thereof. Further, while the embodiments described above may make reference to specific hardware and software components, those skilled in the art will appreciate that different combinations of hardware or software components may also be used and that particular operations described as being implemented in hardware might also be implemented in software or vice versa.

Computer programs incorporating various features of the present disclosure may be encoded and stored on various computer readable storage media; suitable media include magnetic disk or tape, optical storage media such as compact disk (CD) or digital versatile disk (DVD), flash memory, and other non-transitory media. Computer readable media encoded with the program code may be packaged with a compatible electronic device, or the program code may be provided separately from electronic devices (e.g., via Internet download or as a separately packaged computer-readable storage medium).

Thus, although the disclosure has been described with respect to specific embodiments, it will be appreciated that the disclosure is intended to cover all modifications and equivalents within the scope of the following claims.

Examples embodiments of the disclosure are set out in the following numbered clauses.
1. A method of providing instructions for an intervention for an obesity condition in a user, comprising:
   receiving, by one or more processors, one or more measurements of a metabolic system of the user over a measured time period;
   applying, by the one or more processors, the one or more measurements to a function to generate an output for a subsequent time period;
   updating, by the one or more processors, using the output from the function, at least one of a plurality of weights of a digital twin of the user, the plurality of weights corresponding to the metabolic system of the user;
   generating, by the one or more processors, using the digital twin, a metric indicative of an obesity condition in the metabolic system of the user for the subsequent time period;
   identifying, by the one or more processors, an intervention for the obesity condition based on the metric; and
   providing, by the one or more processors, to a user device, an instruction identifying the intervention for the metabolic system of the user.
2. The method of clause 1, further comprising providing, by the one or more processors, an instruction for administering the intervention for the obesity condition to the user.
3. The method of clause 1 or clause 2, further comprising:
   updating, by the one or more processors, the digital twin with information on the administration of the intervention; and
   generating, by the one or more processors, using the digital twin, a second metric of the metabolic system based on the information.
4. The method of any one of clauses 1-3, wherein the one or more measurements of the metabolic system comprise at least one of: glucose excretion, an evaluation of an infradian or circadian rhythm, energy expenditure, physical activity, hormone levels, body weight, body fat percentage, a genetic marker, an evaluation of the gut microbiome, or energy intake.
5. The method of any one of clauses 1-4, wherein the intervention is selected from one or more of the following: a weight loss medication, physical activity, a reduction in energy intake, food choices, cognitive training, behavioral therapy, an optimal mealtime, an exercise routine, and a surgical intervention.
6. The method of claim 5, wherein the weight loss medication is selected from one or more of a GLP-1 receptor agonist, GIP receptor agonists, a lipase inhibitor, an amphetamine, an antidepressant, an opioid medication, and a melanocortin receptor agonist.
7. The method of claim 5, wherein the weight loss medication is a GLP-1 receptor agonist selected from one or more of semaglutide, liraglutide, exenatide, and dulaglutide, or a GIP receptor agonist comprising tirzepatide.
8. The method of any one of clauses 1-7, wherein the updating further comprises updating at least one of the plurality of weights of the digital twin responsive to changes in the one or more measurements applied to the function.
9. The method of clause 8, wherein the updating is in real-time and/or responsive to the changes in the one more measurements.
10. The method of clause 9, wherein the updating is responsive to any one of the following: activity level, meal timing, the time of day, circadian rhythms, and sleep and/or wake timing.
11. The method of any one of clauses 1-10, wherein the function is a filter.
12. The method of clause 11, wherein the filter comprises at least one of a Kalman filter, a particle filter, a sliding window filter, an information filter, an extended Kalman filter, or an unscented Kalman filter.
13. The method of any one of clauses 1-12, further comprising simulating, by the one or more processors, a second metric indicating a condition of the metabolic system of the user in response to the identification of the intervention.
14. The method of clause 13, wherein the second metric comprises a simulation of the user's energy balance, weight loss trajectory, energy intake, energy output, physical activity, glucose excretion, a simulation of a body composition change, a metabolic rate, a weight loss prediction, glucose fluctuations, or energy expenditure.
15. The method of clause 13 or clause 14, further comprising generating a visual output of the second metric to the user device.
16. The method of clause 15, wherein the visual output is updated based on the preferences of the user.
17. The method of clause 16, wherein the preferences of the user comprise any one of the following: metrics of metabolic health, graphical outputs, or visual format of the interface.
18. The method of any one of clauses 15-17, wherein the visual output is an avatar of the digital twin based on a body composition change resulting from the simulated introduction of the intervention, an AI generated image, an infographic, a graphical output, or a metric of metabolic health.
19. The method of any one of clauses 1-18 wherein the digital twin comprises a corresponding plurality of scores determined by the one or more measurements of the user.
20. The method of clause 19, wherein the generating of the metric of metabolic health further comprises determining the metric based on at least one of: (i) an average of the plurality of scores, (ii) a weighted combination of the plurality of scores, or (iii) a comparison with a dataset comprised of a second plurality of scores.
21. The method of any one of clauses 1-20, further comprising generating, by the one or more processors, a plurality of interventions or treatment strategies.
22. The method of clause 21, wherein the user selects an intervention or treatment strategy from the plurality of interventions or treatment strategies; and/or wherein generating, by the one or more processors, a plurality of interventions or treatment strategies comprises the processor generating the plurality of interventions or treatment strategies for user selection; and/or wherein the method further comprises receiving, by the one or more processors, a user selection of an intervention or treatment strategy from the plurality of interventions or treatment strategies.
23. A system for providing instructions for an intervention for an obesity condition in a user, comprising:
   one or more processors, configured to
   receive one or more measurements of a metabolic system of the user over a measured time period;
   apply the one or more measurements to a function to generate an output for a subsequent time period;
   update, using the output from the function, at least one of a plurality of weights of a digital twin of the user, the plurality of weights corresponding to the metabolic system of the user;
   generate, using the digital twin, a metric indicative of an obesity condition in the metabolic system of the user for the subsequent time period;
   identify an intervention for the obesity condition based on the metric; and
   provide to a user device, an instruction identifying the intervention for the metabolic system of the user.
24. The system of clause 23, wherein the one or more processors are configured to provide an instruction for administering the intervention for the obesity condition to the user.
25. The system of clause 23 or clause 24, wherein the one or more processors are configured to:
   update the digital twin with information on the administration of the intervention; and
   generate using the digital twin, a second metric of the metabolic system based on the information.
26. The system of any one of clauses 23-25, wherein the one or more measurements of the metabolic system comprise at least one of: glucose excretion, an evaluation of an infradian or circadian rhythm, energy expenditure, physical activity, hormone levels, body weight, body fat percentage, a genetic marker, an evaluation of the gut microbiome, or energy intake.
27. The system of any one of clauses 23-26, wherein the intervention is selected from one or more of the following: a weight loss medication, physical activity, a reduction in energy intake, food choices, cognitive training, behavioral therapy, an optimal mealtime, an exercise routine, and a surgical intervention.
28. The system of clause 27, wherein the weight loss medication is selected from one or more of a GLP-1 receptor agonist, GIP receptor agonists, a lipase inhibitor, an amphetamine, an antidepressant, an opioid medication, and a melanocortin receptor agonist.
29. The system of clause 28, wherein the weight loss medication is a GLP-1 receptor agonist selected from one or more of semaglutide, liraglutide, exenatide, and dulaglutide, or a GIP receptor agonist comprising tirzepatide.
30. The system of any one of clauses 23-29, wherein the updating further comprises updating at least one of the plurality of weights of the digital twin responsive to changes in the one or more measurements applied to the filter.
31. The system of clause 30, wherein the updating is in real-time and responsive to the changes in the one or more measurements.
32. The system of clause 31, wherein the updating is responsive to any one of the following: the user's activity level, meal timing, the time of day, the user's circadian rhythms, and sleep and/or wake timing.
33. The system of any one of clauses 23-32, wherein the function is a filter.
34. The system of clause 33, wherein the filter comprises at least one of a Kalman filter, a particle filter, a sliding window filter, an information filter, an extended Kalman filter, or an unscented Kalman filter.
35. The system of any one of clauses 23-34, wherein the one or more processors are configured to simulate a second metric indicating a condition of the metabolic system of the user in response to the introduction of the intervention.
36. The system of clause 35, wherein the second metric comprises a simulation of the user's energy balance, weight loss trajectory, energy intake, energy output, physical activity, glucose excretion, a simulation of a body composition change, a metabolic rate, a weight loss prediction, glucose fluctuations, or energy expenditure.
37. The system of clause 35 or clause 36, further comprising generating a visual output of the second metric to the user device.
38. The system of clause 37, wherein the visual output is updated based on the preferences of the user.
39. The system of clause 38, wherein the preferences of the user are selected from: a plurality of metrics of metabolic health, graphical outputs, and the visual format of the interface.
40. The system of any one of clauses 37-39, wherein the visual output is an avatar of the digital twin based on a body composition change resulting from the simulated introduction of the intervention, an AI generated image of the digital twin, an infographic, a graphical output, or a metric of metabolic health.
41. The system of any one of clauses 23-40 wherein the digital twin comprises a corresponding plurality of scores determined by the one or more measurements of the user.
42. The system of clause 41, wherein the generating of the metric of metabolic health further comprises determining the metric based on at least one of: (i) an average of the plurality of scores, (ii) a weighted combination of the plurality of scores, or (iii) a comparison with a dataset comprised of a second plurality of scores.
43. The system of any one of clauses 23-42, further comprising generating, by the one or more processors a plurality of treatment strategies.
44. The system of clause 43, wherein the user selects a treatment strategy from the plurality of treatment strategies.
45. A method of ameliorating an obesity condition in a user in need thereof, comprising:
   administering, to the user, a treatment comprising a digital therapeutic, the digital therapeutic comprising:
   receiving one or more measurements of a metabolic system of the user over a measured time period;
   applying the one or more measurements to a function to generate an output for a subsequent time period;
   updating, using the output from the function, at least one of a plurality of weights of a digital twin of the user, the plurality of weights corresponding to the metabolic system of the user;
   generating, using the digital twin, a metric of the metabolic system of the user for the subsequent time period; and
   identifying an intervention for the obesity condition based on the metric.
46. The method of clause 45, wherein the digital therapeutic comprises obtaining a second metric of the metabolic system of the user subsequent to administering the digital therapeutic.
47. The method of clause 45 or clause 46, wherein administration of the digital therapeutic results in the second metric that is a decrease from the first metric by a first predetermined margin.
48. The method of clause 47, wherein the first metric and the second metric comprise at least one of body weight values or weight efficacy lifestyle questionnaire (WEL) values.
49. The method of clause 47, wherein administration of the digital therapeutic results in the second metric that is (ii) increased from the first metric by a second predetermined margin.
50. The method of clause 49, wherein the first metric and the second metric comprise a computerized performance assessment value for inhibitory control.
51. The method of any one of clauses 45-50, further comprising administering the intervention for the treatment of the obesity condition to the user.
52. The method of any one of clauses 45-51, wherein the digital therapeutic further comprises determining a second intervention for the obesity condition in the user.
53. The method of clause 52, further comprising administering the second intervention for the obesity condition to the user.
54. The method of any one of clauses 45-53, wherein the first metric of the obesity condition is based on a plurality of weights of the digital twin.
55. The method of any one of clauses 45-54, wherein the digital therapeutic further comprises simulating, by one or more processors, a third metric of the metabolic system of the user based on the administration of the intervention to the user.
56. The method of clause 55, wherein the digital therapeutic further comprises generating a visual output of the third metric to a user device.
57. The method of any one of clauses 45-56, wherein the one or more measurements of the metabolic system comprise at least one of: glucose excretion, an evaluation of an infradian or circadian rhythm, energy expenditure, physical activity, hormone levels, body weight, body fat percentage, a genetic marker, an evaluation of the gut microbiome, or energy intake.
58. The method of any one of clauses 45-57, wherein the intervention is selected from one or more of the following: a weight loss medication, physical activity, a reduction in energy intake, food choices, cognitive training, behavioral therapy, an optimal mealtime, an exercise routine, and a surgical intervention.
59. The method of clause 58, wherein the weight loss medication is selected from one or more of a GLP-1 receptor agonist, GIP receptor agonists, a lipase inhibitor, an amphetamine, an antidepressant, an opioid medication, and a melanocortin receptor agonist.
60. The method of clause 59, wherein the weight loss medication is a GLP-1 receptor agonist selected from one or more of semaglutide, liraglutide, exenatide, and dulaglutide, or a GIP receptor agonist comprising tirzepatide.
61. The method of any one of clauses 45-60, wherein the user is receiving treatment for obesity.
62. The method of any one of clauses 45-61, wherein the user is suffering from diabetes, high blood pressure, hypercholesterolemia, fatigue, excess body fat, psychological issues, snoring, shortness of breath, or physical impairments.
63. The method of any one of clauses 45-60, wherein the intervention is administered over a period of 1 day, 5 days, 1 week, 2 weeks, 1 months, 3 months, or 6 months.
64. The method of any one of clauses 45-63, wherein the user is provided with a wearable technology configured to obtain one or more of the measurements; and/or wherein one or more of the measurements are received from a wearable device configured to obtain the one or more of the measurements.
65. The method of any one of clauses 45-64, wherein the user has a BMI greater than or equal to 25, greater than or equal to 30, greater than or equal to 32, or greater than or equal to 35.
66. The method of any one of clauses 45-65, wherein the user has a body fat percentage greater than 20%, greater than 25%, or greater than 30%.
67. The method of any one of clauses 45-66, wherein administration of the digital therapeutic results in a reduction of BMI, body fat percentage, blood glucose, or body weight.
68. The method of any one of clauses 45-67, wherein the first metric indicates the presence of the obesity condition.
69. The method of any one of clauses 46-68, wherein the second metric indicates the absence of the obesity condition.
70. The method of any one of clauses 55-69, wherein the third metric indicates the absence of the obesity condition.
71. The method of any one clauses 45-70, wherein the one or more measurements are physiological measurements.
72. A system for providing instructions for an intervention for an obesity condition in a user, comprising: one or more processors, configured to implement the method of any of clauses 1 to 22.
73. A computer-readable medium storing instructions that, when executed by one or more processors, cause the one or more processors to execute a method of ameliorating an obesity condition in a user in need thereof, the method comprising:
   administering, to the user, a treatment comprising a digital therapeutic, the digital therapeutic comprising:
   receiving one or more measurements of a metabolic system of the user over a measured time period;
   applying the one or more measurements to a function to generate an output for a subsequent time period;
   updating, using the output from the function, at least one of a plurality of weights of a digital twin of the user, the plurality of weights corresponding to the metabolic system of the user;
   generating, using the digital twin, a metric of the metabolic system of the user for the subsequent time period; and
   identifying an intervention for the obesity condition based on the metric.
74. The computer-readable medium of clause 73, wherein the digital therapeutic comprises obtaining a second metric of the metabolic system of the user subsequent to administering the digital therapeutic.
75. The computer-readable medium of clause 73 or clause 74, wherein administration of the digital therapeutic results in the second metric that is a decrease from the first metric by a first predetermined margin.
76. The computer-readable medium of clause 75, wherein the first metric and the second metric comprise at least one of body weight values or weight efficacy lifestyle questionnaire (WEL) values.
77. The computer-readable medium of clause 75, wherein administration of the digital therapeutic results in the second metric that is (ii) increased from the first metric by a second predetermined margin.
78. The computer-readable medium of clause 77, wherein the first metric and the second metric comprise a computerized performance assessment value for inhibitory control.
79. The computer-readable medium of any one of clauses 73-78, wherein the digital therapeutic further comprises determining a second intervention for the obesity condition in the user.
80. The computer-readable medium of any one of clauses 73-79, wherein the first metric of the obesity condition is based on a plurality of weights of the digital twin.
81. The computer-readable medium of any one of clauses 73-80, wherein the digital therapeutic further comprises simulating, by one or more processors, a third metric of the metabolic system of the user based on the administration of the intervention to the user.
82. The computer-readable medium of clause 81, wherein the digital therapeutic further comprises generating a visual output of the third metric to a user device.
83. The computer-readable medium of any one of clauses 73-82, wherein the one or more measurements of the metabolic system comprise at least one of: glucose excretion, an evaluation of an infradian or circadian rhythm, energy expenditure, physical activity, hormone levels, body weight, body fat percentage, a genetic marker, an evaluation of the gut microbiome, or energy intake.
84. The computer-readable medium of any one of clauses 73-83, wherein the intervention is selected from one or more of the following: a weight loss medication, physical activity, a reduction in energy intake, food choices, cognitive training, behavioral therapy, an optimal mealtime, an exercise routine, and a surgical intervention.
85. The computer-readable medium of clause 84, wherein the weight loss medication is selected from one or more of a GLP-1 receptor agonist, GIP receptor agonists, a lipase inhibitor, an amphetamine, an antidepressant, an opioid medication, and a melanocortin receptor agonist.
86. The computer-readable medium of clause 85, wherein the weight loss medication is a GLP-1 receptor agonist selected from one or more of semaglutide, liraglutide, exenatide, and dulaglutide, or a GIP receptor agonist comprising tirzepatide.
87. The computer-readable medium of any one of clauses 73-86, wherein the user is receiving treatment for obesity.
88. The computer-readable medium of any one of clauses 73-87, wherein the user is suffering from diabetes, high blood pressure, hypercholesterolemia, fatigue, excess body fat, psychological issues, snoring, shortness of breath, or physical impairments.
89. The computer-readable medium of any one of clauses 73-88, wherein the intervention is administered over a period of 1 day, 5 days, 1 week, 2 weeks, 1 months, 3 months, or 6 months.
90. The computer-readable medium of any one of clauses 73-89, wherein the user is provided with a wearable technology configured to obtain one or more of the measurements; and/or wherein one or more of the measurements are received from a wearable device configured to obtain the one or more of the measurements.
91. The computer-readable medium of any one of clauses 73-90, wherein the user has a BMI greater than or equal to 25, greater than or equal to 30, greater than or equal to 32, or greater than or equal to 35.
92. The computer-readable medium of any one of clauses 73-91, wherein the user has a body fat percentage greater than 20%, greater than 25%, or greater than 30%.
93. The computer-readable medium of any one of clauses 73-92, wherein administration of the digital therapeutic results in a reduction of BMI, body fat percentage, blood glucose, or body weight.
94. The computer-readable medium of any one of clauses 73-93, wherein the first metric indicates the presence of the obesity condition.
95. The computer-readable medium of any one of clauses 73-94, wherein the second metric indicates the absence of the obesity condition.
96. The computer-readable medium of any one of clauses 55-95, wherein the third metric indicates the absence of the obesity condition.
97. The computer-readable medium of any one clauses 73-96, wherein the one or more measurements are physiological measurements.
98. A computer-implemented method comprising:
   receiving one or more measurements of a metabolic system of the user over a measured time period;
   applying the one or more measurements to a function to generate an output for a subsequent time period;
   updating, using the output from the function, at least one of a plurality of weights of a digital twin of the user, the plurality of weights corresponding to the metabolic system of the user;
   generating, using the digital twin, a metric of the metabolic system of the user for the subsequent time period; and
   identifying an intervention for the obesity condition based on the metric.
99. The method of clause 98, wherein the method comprises obtaining a second metric of the metabolic system of the user subsequent to identifying the intervention.
100. The method of clause 98 or clause 99, wherein the method results in the second metric that is a decrease from the first metric by a first predetermined margin.
101. The method of clause 100, wherein the first metric and the second metric comprise at least one of body weight values or weight efficacy lifestyle questionnaire (WEL) values.
102. The method of clause 199, wherein the method results in the second metric that is increased from the first metric by a second predetermined margin.
103. The method of clause 102, wherein the first metric and the second metric comprise a computerized performance assessment value for inhibitory control.
104. The method of any one of clauses 98-103, further comprising administering the intervention for the treatment of the obesity condition to the user.
105. The method of any one of clauses 98-104, wherein the method further comprises determining a second intervention for the obesity condition in the user.
106. The method of clause 105, further comprising administering the second intervention for the obesity condition to the user.
107. The method of any one of clauses 98-106, wherein the first metric of the obesity condition is based on a plurality of weights of the digital twin.
108. The method of any one of clauses 98-107, wherein the method further comprises simulating a third metric of the metabolic system of the user based on the administration of the intervention to the user.
109. The method of clause 108, wherein the digital therapeutic further comprises generating a visual output of the third metric to a user device.
110. The method of any one of clauses 98-109, wherein the one or more measurements of the metabolic system comprise at least one of: glucose excretion, an evaluation of an infradian or circadian rhythm, energy expenditure, physical activity, hormone levels, body weight, body fat percentage, a genetic marker, an evaluation of the gut microbiome, or energy intake.
111. The method of any one of clauses 98-110, wherein the intervention is selected from one or more of the following: a weight loss medication, physical activity, a reduction in energy intake, food choices, cognitive training, behavioral therapy, an optimal mealtime, an exercise routine, and a surgical intervention.
112. The method of clause 111, wherein the weight loss medication is selected from one or more of a GLP-1 receptor agonist, GIP receptor agonists, a lipase inhibitor, an amphetamine, an antidepressant, an opioid medication, and a melanocortin receptor agonist.
113. The method of clause 112, wherein the weight loss medication is a GLP-1 receptor agonist selected from one or more of semaglutide, liraglutide, exenatide, and dulaglutide, or a GIP receptor agonist comprising tirzepatide.
114. The method of any one of clauses 98-113, wherein the user is receiving treatment for obesity.
115. The method of any one of clauses 98-114, wherein the user is suffering from diabetes, high blood pressure, hypercholesterolemia, fatigue, excess body fat, psychological issues, snoring, shortness of breath, or physical impairments.
116. The method of any one of clauses 98-115, wherein the intervention is administered over a period of 1 day, 5 days, 1 week, 2 weeks, 1 months, 3 months, or 6 months.
117. The method of any one of clauses 98-116, wherein the user is provided with a wearable technology configured to obtain one or more of the measurements; and/or wherein one or more of the measurements are received from a wearable device configured to obtain the one or more of the measurements.
118. The method of any one of clauses 98-117, wherein the user has a BMI greater than or equal to 25, greater than or equal to 30, greater than or equal to 32, or greater than or equal to 35.
119. The method of any one of clauses 98-118, wherein the user has a body fat percentage greater than 20%, greater than 25%, or greater than 30%.
120. The method of any one of clauses 98-119, wherein administration of the digital therapeutic results in a reduction of BMI, body fat percentage, blood glucose, or body weight.
121. The method of any one of clauses 98-120, wherein the first metric indicates the presence of the obesity condition.
122. The method of any one of clauses 99-121, wherein the second metric indicates the absence of the obesity condition.
123. The method of any one of clauses 55-122, wherein the third metric indicates the absence of the obesity condition.
124. The method of any one clauses 98-123, wherein the one or more measurements are physiological measurements.
125. A computer-readable medium storing instructions that, when executed by one or more processors, cause the one or more processors to execute a method of ameliorating an obesity condition in a user in need thereof, the method comprising: administering, to the user, a treatment comprising a digital therapeutic, the digital therapeutic comprising the method of any of clauses 98-125.
126. A system for ameliorating an obesity condition in a user in need thereof, the system comprising one or more processor and a computer readable medium according to any one of clauses 73-97 or 125.

## Claims

1. A method of providing instructions for an intervention for an obesity condition in a user, comprising:
receiving, by one or more processors, one or more measurements of a metabolic system of the user over a measured time period;
applying, by the one or more processors, the one or more measurements to a function to generate an output for a subsequent time period;
updating, by the one or more processors, using the output from the function, at least one of a plurality of weights of a digital twin of the user, the plurality of weights corresponding to the metabolic system of the user;
generating, by the one or more processors, using the digital twin, a metric indicative of an obesity condition in the metabolic system of the user for the subsequent time period;
identifying, by the one or more processors, an intervention for the obesity condition based on the metric; and
providing, by the one or more processors, to a user device, an instruction identifying the intervention for the metabolic system of the user.

2. The method of claim 1, further comprising:
(i) providing, by the one or more processors, an instruction for administering the intervention for the obesity condition to the user; and/or
(ii) updating, by the one or more processors, the digital twin with information on the administration of the intervention; and
generating, by the one or more processors, using the digital twin, a second metric of the metabolic system based on the information.

3. The method of claim 1 or claim 2, wherein the one or more measurements of the metabolic system comprise at least one of: glucose excretion, an evaluation of an infradian or circadian rhythm, energy expenditure, physical activity, hormone levels, body weight, body fat percentage, a genetic marker, an evaluation of the gut microbiome, or energy intake; and/or wherein the intervention is selected from one or more of the following: a weight loss medication, physical activity, a reduction in energy intake, food choices, cognitive training, behavioral therapy, an optimal mealtime, an exercise routine, and a surgical intervention.

4. The method of claim 3, wherein the weight loss medication is selected from one or more of a GLP-1 receptor agonist, GIP receptor agonists, a lipase inhibitor, an amphetamine, an antidepressant, an opioid medication, and a melanocortin receptor agonist, or wherein the weight loss medication is a GLP-1 receptor agonist selected from one or more of semaglutide, liraglutide, exenatide, and dulaglutide, or a GIP receptor agonist comprising tirzepatide.

5. The method of any one of claims 1-4, wherein the updating further comprises updating at least one of the plurality of weights of the digital twin responsive to changes in the one or more measurements applied to the function, and/or wherein the updating is in real-time and/or responsive to the changes in the one more measurements.

6. The method of claim 5, wherein the updating is responsive to any one of the following: activity level, meal timing, the time of day, circadian rhythms, and sleep and/or wake timing.

7. The method of any one of claims 1-6, wherein the function is a filter, or wherein the function is a filter comprising at least one of a Kalman filter, a particle filter, a sliding window filter, an information filter, an extended Kalman filter, or an unscented Kalman filter.

8. The method of any one of claims 1-7, further comprising simulating, by the one or more processors, a second metric indicating a condition of the metabolic system of the user in response to the identification of the intervention;
optionally wherein the second metric comprises a simulation of the user's energy balance, weight loss trajectory, energy intake, energy output, physical activity, glucose excretion, a simulation of a body composition change, a metabolic rate, a weight loss prediction, glucose fluctuations, or energy expenditure.

9. The method of claim 8, further comprising generating a visual output of the second metric to the user device,
optionally wherein:
(i) the visual output is updated based on the preferences of the user, optionally wherein the preferences of the user comprise any one of the following: metrics of metabolic health, graphical outputs, or visual format of the interface; and/or
(ii) the visual output is an avatar of the digital twin based on a body composition change resulting from the simulated introduction of the intervention, an AI generated image, an infographic, a graphical output, or a metric of metabolic health.

10. The method of any one of claims 1-9 wherein the digital twin comprises a corresponding plurality of scores determined by the one or more measurements of the user,
optionally wherein the generating of the metric of metabolic health further comprises determining the metric based on at least one of: (i) an average of the plurality of scores, (ii) a weighted combination of the plurality of scores, or (iii) a comparison with a dataset comprised of a second plurality of scores.

11. The method of any one of claims 1-10, further comprising generating, by the one or more processors, a plurality of interventions or treatment strategies, optionally wherein the user selects an intervention from the plurality of interventions or treatment strategies.

12. A system for providing instructions for an intervention for an obesity condition in a user, comprising: one or more processors, configured to implement the method of any preceding claim.

13. A computer-readable medium storing instructions that, when executed by one or more processors, cause the one or more processors to execute a method of ameliorating an obesity condition in a user in need thereof, the method comprising:
administering, to the user, a treatment comprising a digital therapeutic, the digital therapeutic comprising:
receiving one or more measurements of a metabolic system of the user over a measured time period;
applying the one or more measurements to a function to generate an output for a subsequent time period;
updating, using the output from the function, at least one of a plurality of weights of a digital twin of the user, the plurality of weights corresponding to the metabolic system of the user;
generating, using the digital twin, a metric of the metabolic system of the user for the subsequent time period; and
identifying an intervention for the obesity condition based on the metric.

14. The computer-readable medium of claim 13, wherein:
(i) the digital therapeutic comprises obtaining a second metric of the metabolic system of the user subsequent to administering the digital therapeutic; and/or
(ii) administration of the digital therapeutic results in the second metric that is a decrease from the first metric by a first predetermined margin, optionally wherein the first metric and the second metric comprise at least one of body weight values or weight efficacy lifestyle questionnaire (WEL) values; and/or
(iii) administration of the digital therapeutic results in the second metric that is increased from the first metric by a second predetermined margin, optionally wherein the first metric and the second metric comprise a computerized performance assessment value for inhibitory control.

15. The computer-readable medium of claim 13 or claim 14, wherein the digital therapeutic further comprises determining a second intervention for the obesity condition in the user.

16. The computer-readable medium of any one of claims 13 to 15, wherein the first metric of the obesity condition is based on a plurality of weights of the digital twin.

17. The computer-readable medium of any one of claims 13 to 16, wherein the digital therapeutic further comprises simulating, by one or more processors, a third metric of the metabolic system of the user based on the administration of the intervention to the user, optionally wherein the digital therapeutic further comprises generating a visual output of the third metric to a user device.

18. The computer-readable medium of any one of claims 13-15, wherein the one or more measurements of the metabolic system comprise at least one of: glucose excretion, an evaluation of an infradian or circadian rhythm, energy expenditure, physical activity, hormone levels, body weight, body fat percentage, a genetic marker, an evaluation of the gut microbiome, or energy intake; and/or
wherein the intervention is selected from one or more of the following: a weight loss medication, physical activity, a reduction in energy intake, food choices, cognitive training, behavioral therapy, an optimal mealtime, an exercise routine, and a surgical intervention,
optionally wherein the weight loss medication is selected from one or more of a GLP-1 receptor agonist, GIP receptor agonists, a lipase inhibitor, an amphetamine, an antidepressant, an opioid medication, and a melanocortin receptor agonist, or wherein the weight loss medication is a GLP-1 receptor agonist selected from one or more of semaglutide, liraglutide, exenatide, and dulaglutide, or a GIP receptor agonist comprising tirzepatide.

19. The computer-readable medium of any one of claims 13-18, wherein the user is receiving treatment for obesity; and/or
wherein the user is suffering from diabetes, high blood pressure, hypercholesterolemia, fatigue, excess body fat, psychological issues, snoring, shortness of breath, or physical impairments; and/or
wherein the intervention is administered over a period of 1 day, 5 days, 1 week, 2 weeks, 1 months, 3 months, or 6 months; and/or
wherein the user is provided with a wearable technology configured to obtain one or more of the measurements; and/or
wherein the one or more measurements are physiological measurements; and/or
wherein one or more of the measurements are received from a wearable device configured to obtain the one or more of the measurements; and/or
wherein the user has a BMI greater than or equal to 25, greater than or equal to 30, greater than or equal to 32, or greater than or equal to 35; and/or
wherein the user has a body fat percentage greater than 20%, greater than 25%, or greater than 30%; and/or
wherein administration of the digital therapeutic results in a reduction of BMI, body fat percentage, blood glucose, or body weight; and/or
wherein the first metric indicates the presence of the obesity condition; and/or
wherein the second metric indicates the absence of the obesity condition; and/or
wherein the third metric indicates the absence of the obesity condition.
